# EUROPÄISCHE PATENTSCHRIFT

(11) **EP 2 718 339 B1**
(45) Veröffentlichungstag und Bekanntmachung des Hinweises auf die Patenterteilung: **07.01.2015**
(21) Anmeldenummer: 12719711.9
(22) Anmeldetag: 08.05.2012
(51) Int. Cl.: C08F 265/02, C08F 265/04, A61K 8/91, A61Q 5/00, C08F 2/22, C09D 7/00, A61Q 5/02, A61Q 5/10, A61Q 5/12, A61Q 5/06

(54) **ASSOZIATIVVERDICKER AUF BASIS VON (METH)ACRYLAT**
ASSOCIATIVE THICKENERS BASED ON (METH)ACRYLATE
ÉPAISSISSANT ASSOCIATIF À BASE DE (MÉTH)ACRYLATE

(30) Priorität: 07.06.2011 EP 11168922
(43) Veröffentlichungstag der Anmeldung: 16.04.2014
(73) Patentinhaber: BASF SE, 67056 Ludwigshafen (DE)
(72) Erfinder: ANGEL, Maximilian, 67105 Schifferstadt (DE); HÖSSEL, Peter, 67105 Schifferstadt (DE)
(86) Internationale Anmeldenummer: PCT/EP2012/058419
(87) Internationale Veröffentlichungsnummer: WO 2012/168016

(56) Entgegenhaltungen:
- EP-A1- 0 549 280
- WO-A1-2009/062994
- DE-A1- 4 318 033

## Beschreibung

Die Erfindung betrifft ein verbessertes Verfahren zur Herstellung von in wässrigem Medium löslichen Polymeren auf (Meth)acrylat-Basis, die sich vor allem als assoziative und nichtassoziative Verdicker einsetzen lassen.

Verdickungsmittel dienen dazu, die Viskosität fließfähiger Zusammensetzungen zu erhöhen. Vor allem in der Kosmetik übliche Verdickungsmittel sind beispielsweise hochmolekulare Polyacrylsäuren (INCI-Bezeichnung: Carbomer), die in ihrer neutralisierten Form als wasserlösliche Verdickungsmittel eingesetzt werden.

Als Assoziativverdicker werden Polymere bezeichnet, die zwar mit ionischen Gruppen wie Carboxylgruppen wasserlöslich gemacht werden, deren verdickende Wirkung aber nicht auf ihrem hohen Molekulargewicht beruht, sondern darauf, dass die in geringen Mengen in die Polymere eingebauten langkettigen aliphatischen Seitengruppen assoziieren. Diese Polymere lassen sich beispielsweise als Lösungs- oder Emulsionspolymerisate herstellen und aus diesen Form heraus anwenden. Polymere dieser Art sind beispielsweise in DE 3404537 und EP 11806 (Dow Chemicals) beschrieben.

Die meisten der als Verdicker bekannten Polymere haben zwar sehr gute verdickende Eigenschaften, gleichzeitig aber auch Nachteile, wenn außer der verdickenden Wirkung weitere Anforderungen an sie gestellt werden.

Insbesondere in der Kosmetik ist es häufig erwünscht, dass Polymere gleichzeitig mehrere Aufgaben erfüllen, beispielsweise zusätzlich zur verdickenden Eigenschaft auch filmbildende Eigenschaften aufweisen. Die Filmbildung in der Haarkosmetik dient in erster Linie dazu, das Haar zu festigen oder besser formen zu können.

Bekannte, häufig auf Ethylacrylat basierende Emulsionspolymere sind zwar gute Filmbildner, häufig aber zu klebrig und weisen deshalb auch keine zufriedenstellenden festigenden Eigenschaften auf. Die von ihnen gebildeten Filme sind zu weich und verleihen dem Haar keine zusätzliche Performance.

Eine Aufgabe der vorliegenden Erfindung war es, ein wirtschaftliches Verfahren anzugeben, mit dem polymere assoziative und nicht assoziative Verdickungsmittel, die sich für kosmetische Anwendungen eignen, hergestellt werden können, sowie polymere assoziative und nichtassoziative Verdickungsmittel zur Verfügung zu stellen, die außer ihrer Verdickungswirkung eine vorteilhafte Anwendung als Filmbildner aus dem wässrigen Medium heraus und möglichst weitere Vorteile bei der Verwendung in kosmetischen Anwendungen bieten.

Gelförmige Präparate für die Kosmetik sollen möglichst viele der folgenden Eigenschaften in sich vereinen:
- die erhaltenen Gele sollen möglichst klar sein,
- die erhaltenen Gele sollen sich leicht im Haar verteilen lassen und diesem guten Halt verleihen, was sich besonders gut durch Gele mit thixotropen Eigenschaften erzielen lässt,
- die erhaltenen Gele sollen selbst über filmbildende Eigenschaften verfügen und so zur Festigung der Haare beitragen,
- die erhaltenen Gele sollen über konditionierende Eigenschaften verfügen und die sensorischen Eigenschaften des Haars verbessern, z. B. ihm Geschmeidigkeit und Glanz verleihen und nach dem Trocken nicht oder nur gering klebrig sein,
- das mit den erhaltenen Gelen behandelte Haar soll eine gute Nasskämmbarkeit aufweisen (somit lässt sich das frisch behandelte Haar leicht mit dem Kamm in Form bringen, um die gewünschte Frisur zu formen),
- die zur Gelherstellung verwendeten Polymere sollen es erlauben, dass in möglichst allen kosmetisch akzeptablen pH-Bereichen, speziell im pH-Bereich von etwa 5 bis 9 Gele formuliert werden können,
- die zur Gelherstellung verwendeten Polymere sollen die Formulierung von Gelen erlauben, deren Eigenschaften über den pH-Wert schaltbar sind,
- die zur Gelherstellung verwendeten Polymere sollen gemeinsam mit handelsüblichen Verdickern formulierbar sein.

Gegenstand der vorliegenden Erfindung ist ein Verfahren zur Herstellung von Polymerisaten P umfassend ein Gemisch aus wenigstens einem Polymer A und wenigstens einem Polymer B, wobei
A) Polymer A in einpolymerisierter Form umfasst
   a1) 30 bis 70 Gew.-% C₁-C₄-Alkyl-(Meth)acrylat,
   a2) 30 bis 70 Gew.-% (Meth)acrylsäure,
   a3) 0 bis 20 Gew.-% C₈-C₃₀-Alkylsubstituierte Monomere,
   a4) 0 bis 20 Gew.-% weitere von □a1) bis □a3) verschiedene Monomere,
   wobei sich die Mengen von □1) bis □a4) zu 100 Gew.-% addieren,
   und
B) Polymer B in einpolymerisierter Form umfasst
   b1) wenigstens ein Monomer □b1), das wenigstens eine Amidgruppe trägt, und
   b2) gegebenenfalls weitere von b1) verschiedene Monomere □b2),
dadurch gekennzeichnet, dass die Monomere eines der Polymere A oder B teilweise oder nahezu vollständig polymerisiert werden und dann die Monomere des jeweils anderen Polymers hinzugegeben werden, wonach anschließend das jeweils andere Polymer und das teilweise polymerisierte Polymer A oder B oder das nahezu vollständig polymerisierte Polymer A oder B unter Bildung des Gemischs aus Polymer A und Polymer B auspolymerisiert werden.

Unter "Polymerisation von Polymer A" bzw. "Polymerisation von Polymer B" wird die Polymerisation der Monomere a1) bis a4) bzw. b1) und b2) verstanden.

Der Ausdruck "das jeweils andere Polymer" hat zwei Bedeutungen: Zum Einen bedeutet der Ausdruck das Polymer A, wenn bereits die Polymerisation von Polymer B teilweise oder nahezu vollständig erfolgt ist, und zum Anderen das Polymer B, wenn die Polymerisation von Polymer A teilweise oder nahezu vollständig erfolgt ist.

Aus dem Stand der Technik sind zahlreiche Polymere bekannt, die durch Polymerisation in Gegenwart von Polyvinylpyrrolidon (PVP) hergestellt werden. Zu nennen sind hier beispielsweise DE 3818868 (Polystyrol in PVP), DE 19710215 (Poly-Vinylformamid in PVP), DE 2050248 (Vinylchlorid-Vinylether-Copolymere in PVP), DE 10048888 (Poly-Vinylacetat in PVP), DE 10311616 (Poly-Vinylformamid-g-PVP) und DE 4318033 (Methylmethacrylat-Methacrylsäure-Tensid in PVP).

**Die** DE 2924663 beschreibt ein Verfahren zur Herstellung von wässrigen Dispersionen aus wasserlöslichen Polymermassen. Dabei werden wasserlösliche Monomere wie beispielsweise Acrylsäure, Acrylamid oder Dimethylaminoethylmethacrylat in Gegenwart von Polymeren wie Polyethern, Polyvinylpyrrolidon oder Polyvinylacetat polymerisiert.

Des Weiteren beschreibt die EP 0 549 280 ein Verfahren zur Herstellung von Pfropfpolymeren, wobei ein sekundärer Cersalzkatalysator als starkes Oxidationsmittel verwendet wird.

### Polymer A

### a1) C₁-C₄-Alkyl-(Meth)acrylat

Polymer A umfasst in einpolymerisierter Form 30 bis 70 Gew.-%, bevorzugt 40 bis 60 Gew.-%, besonders bevorzugt 45 bis 55 Gew.-% C₁-C₄-Alkyl-(Meth)acrylat.

Das C₁-C₄-Alkyl-(Meth)acrylat a1) ist bevorzugt ausgewählt aus Methyl(meth)acrylat, Ethyl(meth)acrylat, n-Propyl(meth)acrylat, iso-Propyl(meth)acrylat, n-Butyl(Meth)acrylat, isoButyl(meth)acrylat, sec-Butyl(meth)acrylat, tert.-Butyl(meth)acrylat und beliebigen Mischungen daraus.

Bevorzugt besteht a1) zu wenigstens 50 Gew.-%, besonders bevorzugt zu wenigstens 80 Gew.-% und insbesondere zu wenigstens 90 Gew.-% aus Ethylacrylat. In einer Ausführungsform der Erfindung umfasst oder ist a1) Ethylacrylat.

Die Monomere a1) sind in den erfindungsgemäß hergestellten Polymeren A) in einer Menge im Bereich von 30-70 Gew.-%, bevorzugt im Bereich von 40-60 Gew.-% einpolymerisiert, bezogen auf die Gesamtmenge aller in den Polymeren A) einpolymerisierten Monomere.

### a2) (Meth)Acrylsäure

Polymer A umfasst in einpolymerisierter Form 30 bis 70 Gew.-%, bevorzugt 35 bis 59 Gew.-%, besonders bevorzugt 40 bis 54 Gew.-% (Meth)acrylsäure.

Der Begriff "(Meth)acrylsäure" umfasst vorliegend Acrylsäure, Methacrylsäure sowie Mischungen daraus. Der Begriff (Meth)acrylsäure umfasst dabei jeweils sowohl die neutrale als auch die neutralisierte, anionische Form der (Meth)acrylsäure.

Bevorzugt besteht a2) zu wenigstens 50 Gew.-%, besonders bevorzugt zu wenigstens 80 Gew.-% und insbesondere zu wenigstens 90 Gew.-% aus Methacrylsäure. In einer Ausführungsform der Erfindung umfasst oder ist a2) Methacrylsäure.

Die Monomere a2) sind in den erfindungsgemäß hergestellten Polymeren A) in einer Menge im Bereich von 30-70 Gew.-%, bevorzugt im Bereich von 40-60 Gew.-% einpolymerisiert, bezogen auf die Gesamtmenge aller in den Polymeren A) einpolymerisierten Monomeren.

### a3) C₈-C₃₀-Alkylsubstituierte Monomere

Polymer A umfasst in einpolymerisierter Form 0 bis 20 Gew.-%, bevorzugt 0,1 bis 10 Gew.-%, besonders bevorzugt 0,5 bis 7,5 Gew.-% C₈-C₃₀-Alkylsubstituierte Monomere. Die C₈-C₃₀-Alkylsubstituierten Monomere a3) sind bevorzugt ausgewählt aus Monomeren der allgemeinen Formeln a3.1) und a3.2) 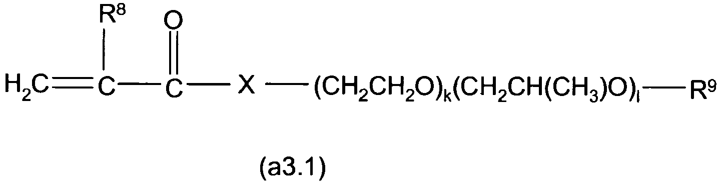 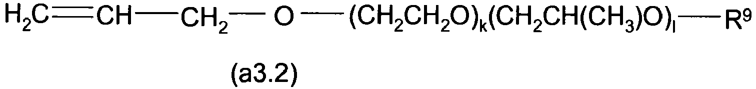 wobei
k und I unabhängig voneinander ganze Zahlen im Bereich von 0 bis 1000 sind und die Summe k+I mindestens gleich 5 ist, R⁸ Wasserstoff oder or C₁-C₄-Alkyl, bevorzugt Methyl, ist R⁹ C₈-C₃₀-Alkyl, C₈-C₃₀-Alkenyl oder C₈-C₃₀ Alkylaryl, ist und X O oder NR¹⁰ ist, wobei R¹⁰ ausgewählt ist aus H, Alkyl, Alkenyl, Cycloalkyl, Heterocycloalkyl, Aryl oder Hetaryl.

In einer bevorzugten Ausführungsform der Erfindung sind die Monomere c) ausgewählt aus C₈-C₃₀-Alkylsubstituierten Monomeren.

Unmittelbar an X in a3.1) bzw. an O in a3.2) schließt entweder wenigstens ein Ethylenoxidrest (EO) oder wenigstens ein Propylenoxidrest (PO) an. Bevorzugt schließt unmittelbar an X und O wenigstens ein EO an.

In einer Ausführungsform der Erfindung sind die Monomere a3.1) Ester der (Meth)acrylsäure, also R⁸ in Formel (a3.1) ist H oder bevorzugt CH₃, mit alkoxylierten Alkoholen.

Geeignete alkoxylierte Alkohole sind beispielsweise die alkoxylierten
- linearen Alkohole aus natürlichen Quellen oder aus der Ziegler-Aufbaureaktion von Ethylen in Gegenwart von Aluminiumalkyl-Katalysatoren. Beispiele für geeignete lineare Alkohole sind lineare C₈-C₃₀-Alkohole, insbesondere C₁₂-C₃₀-Alkohole. Als besonders bevorzugte Alkohole seien genannt: n-Dodecanol, n-Tetradecanol, n- Hexadecanol, n-Octadecanol, n-Eicosanol, n-Docosanol, n-Tetracosanol, n-Hexacosanol, n-Octacosanol, und/oder n-Triacontanol, sowie Gemische der vorstehend genannten Alkohole, beispielsweise NAFOL® Typen wie NAFOL® 22+ (Sasol).
- Oxoalkohole wir bespielsweise Isooctanol, Isononanol, Isodecanol, Isoundecanol, Isotridecanol (beispielsweise Exxal®-Typen 7, 8, 9, 10, 11, 13).
- Alkohole, die in 2-Stellung verzweigt sind; hierbei handelt es sich um die dem Fachmann bekannten Guerbetalkohole, die durch Dimerisierung von primären Alkoholen über die so genannte Guerbetreaktion zugänglich sind. Als besonders bevorzugte Alkohole seien hier genannt: Isofol®12 (Sasol), Rilanit®G16 (Cognis).
- Alkohole, die durch die Friedel-Crafts-Alkylierung mit oligomerisierten Olefinen erhalten werden und die dann neben einem gesättigten Kohlenwasserstoffrest einen aromatischen Ring enthalten. Als besonders bevorzugte Alkohole seien hier genannt: i-Octylphenol und i-Nonylphenol.
- Alkohole der allgemeinen Formel (4) der EP 761780 A2, S.4 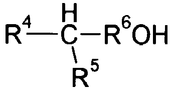 oder Alkohole der allgemeinen Formel (5) der EP 761780 A2, S.4 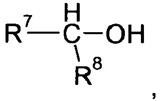 wobei
- R⁴, R⁵, R⁷ und R⁸ unabhängig voneinander die in EP 761780 A2, S.4, Zeilen 45 bis 58 beschriebene Bedeutung haben; bevorzugt sind R⁴, R⁵, R⁷ und R⁸ unabhängig voneinander Alkylreste mit wenigstens 4 Kohlenstoffatomen und die Gesamtzahl der Kohlenstoffatome der Alkohole beträgt höchstens 30,
- R⁶ ein Alkylenrest wie beispielsweise -CH₂-, -CH₂-CH₂-, -CH₂-CH(CH₃)-;

Beispielsweise sei hier 2-Decyl-1-tetradecanol als geeigneter Alkohol genannt.

In einer Ausführungsform ist wenigstens ein a3) ein (Meth)acrylsäureester eines Gemisches ethoxylierter C₈-C₃₀-, bevorzugt C₁₂-C₃₀-, insbesondere C₁₆-C₂₂-Fettalkohole.

In einer Ausführungsform ist wenigstens ein a3) ein (Meth)acrylsäureester eines Gemisches ethoxylierter C₈-C₃₀-, bevorzugt C₁₂-C₃₀-, insbesondere C₁₆-C₂₂-Fettalkohole, wobei die ethoxylierten Alkohole jeweils 20 bis 30 EO-Reste umfassen.

In einer Ausführungsform ist wenigstens ein a3) ein (Meth)acrylsäureester eines Gemisches ethoxylierter C₁₂-C₁₈-Fettalkohole, wobei die ethoxylierten Alkohole jeweils 10 bis 30 EO-Reste umfassen.

In einer Ausführungsform der Erfindung umfasst oder ist a3) ein Methacrylsäureester eines mit 25 Mol Ethylenoxid ethoxylierten C₁₆-C₁₈-Fettalkoholes (auch als "C₁₆₋₁₈-Alkyl-PEG1100 - methacrylate" bezeichnet).

Kommerziell erhältlich sind solche C₁₆₋₁₈-Alkyl-PEG1100 - methacrylate beispielsweise als Plex®6877-O (25 Gew.-%ige Zubereitung in Methylmethacrylat) oder Lutencryl®250 (50 Gew.-%ige Lösung in Methacrylsäure) oder VISIOMER® C18 PEG 1105 MA.

Weitere geeignete Monomere a3) sind Verbindungen der allgemeinen Formel a3.1), wobei R⁸ gleich H oder bevorzugt Methyl, X gleich O, k und I gleichzeitig Null und R⁹ C₈-C₂₀- Alkylaryl oder bevorzugt C₈-C₂₀- Alkyl.

Beispiele für solche Monomere a3) sind n-Octyl(meth)acrylat, 1,1,3,3-Tetramethylbutyl(meth)acrylat, 2-Ethylhexyl(meth)acrylat, n-Nonyl(meth)acrylat, n-Decyl(meth)acrylat, Isodecyl(meth)acrylat, n-Undecyl(meth)acrylat, Isoundecyl(meth)acrylat, Dodecyl(meth)acrylat, Tridecyl(meth)acrylat, Myristyl(meth)acrylat, Pentadecyl(meth)acrylat, Palmityl(meth)acrylat, Heptadecyl(meth)acrylat, Stearyl(meth)acrylat, Lauryl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat und Mischungen davon.

Die Monomere a3) sind in den erfindungsgemäß hergestellten Polymeren A) in einer Menge im Bereich von 0-20 Gew.-%, bevorzugt im Bereich von 0,1-10 Gew.-% einpolymerisiert, bezogen auf die Gesamtmenge aller in den Polymeren A) einpolymerisierten Monomere.

### a4) von □a1) bis Da3) verschiedene Monomere

Polymer A umfasst in einpolymerisierter Form 0 bis 20 Gew.-%, bevorzugt 0 bis 3 Gew.-%, besonders bevorzugt weniger als 0,1 Gew.-% weitere von □a1) bis □a3) verschiedene Monomere.

Unter "von □a1) bis Da3) verschiedene Monomere" sind vorliegend alle diejenigen polymerisierbaren Verbindungen a4) zu verstehen, die von a1) bis a3) verschieden sind und die in ihrer einpolymerisierten Form kosmetisch akzeptabel sind.

### Polymer B

Das von Polymer A verschiedene Polymer B umfasst in einpolymerisierter Form
b1) wenigstens ein Monomer □b1), das wenigstens eine Amidgruppe trägt, und
b2) gegebenenfalls weitere von b1) verschiedene Monomere □b2).

Bevorzugt sind die Monomere b1) ausgewählt unter primären Amiden α,β-ethylenisch ungesättigter Monocarbonsäuren, N-Vinylamiden gesättigter Mo-nocarbonsäuren, N-Vinyllactamen, N-Alkyl- und N,N-Dialkylamiden α,β-ethylenisch ungesättigter Monocarbonsäuren und Mischungen davon.

Bevorzugte Monomere b1) sind N-Vinyllactame und deren Derivate, die z. B. einen oder mehrere Alkylsubstituenten wie Methyl, Ethyl, n-Propyl, Isopropyl, n-Butyl, sek. Butyl, tert.-Butyl etc. aufweisen können. Dazu zählen z. B. N-Vinylpyrrolidon, N Vinylpiperidon, N-Vinylcaprolactam, N-Vinyl-5-methyl-2-pyrrolidon, N Vinyl 5 ethyl 2 pyrrolidon, N-Vinyl-6-methyl-2-piperidon, N-Vinyl-6-ethyl-2-piperidon, N-Vinyl-7-methyl-2-caprolactam und N-Vinyl-7-ethyl-2-caprolactam.

Geeignet als b1) sind auch N-Alkyl- und N,N-Dialkylamide α,β-ethylenisch ungesättigter Monocarbonsäuren, die zusätzlich zu dem Carbonyl-Kohlenstoffatom der Amidgruppe höchstens 7 weitere Kohlenstoffatome aufweisen wie N-Methyl(meth)acrylamid, N Ethyl(meth)acrylamid, N-Propyl(meth)acrylamid, N-(n-Butyl)(meth)acrylamid, N tert Butyl(meth)acrylamid, n-Pentyl(meth)acrylamid, n-Hexyl(meth)acrylamid, n Heptyl(meth)acrylamid, N,N-Dimethyl(meth)acrylamid, N,N-Diethyl(meth)acrylamid, Piperidinyl(meth)acrylamid, Morpholinyl(meth)acrylamid und Mischungen davon.

Geeignet als b1) sind auch N-C₈-C₃₀-Alkyl- und N-(C₁-C₃₀)alkyl-N-(C₈-C₃₀)alkylamide wie n-Octyl(meth)acrylamid,1,1,3,3-Tetramethylbutyl(meth)acrylamid, 2-Ethylhexyl(meth)acrylamid, n-Nonyl(meth)acrylamid, n-Decyl(meth)acrylamid, n Undecyl(meth)acrylamid, Tridecyl(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arachinyl(meth)acrylamid, Behenyl(meth)acrylamid, Lignocerenyl(meth)acrylamid, Cerotinyl(meth)acrylamid, Melissyl(meth)acrylamid, Palmitoleinyl(meth)acrylamid, Oleyl(meth)acrylamid, Linolyl(meth)acrylamid, Linolenyl(meth)acrylamid, Stearyl(meth)acrylamid, Lauryl(meth)acrylamid, N-Methyl-N (n-octyl)(meth)acrylamid, N,N-Di-(n-octyl)(meth)acrylamid und Mischungen davon.

Als Monomere b1) geeignete offenkettige N-Vinylamidverbindungen sind beispielsweise N-Vinylformamid, N-Vinyl-N-methylformamid, N-Vinylacetamid, N Vinyl N methylacetamid, N-Vinyl-N-ethylacetamid, N-Vinylpropionamid, N Vinyl N methylpropionamid, N-Vinyl-butyramid und Mischungen davon, wobei N-Vinylformamid bevorzugt ist.

Besonders bevorzugte Monomere b1) sind N-Vinylpyrrolidon, N-Vinylcaprolactam, N-Vinylformamid, Acrylamid und Methacrylamid. In einer Ausführungsform der Erfindung ist oder umfasst b1) Methacrylamid.

Unter "von b1) verschiedene Monomere b2)" sind vorliegend alle diejenigen mit b1) polymerisierbaren Verbindungen zu verstehen, die von b1) verschieden sind und die in ihrer einpolymerisierten Form kosmetisch akzeptabel sind.

In einer Ausführungsform der Erfindung umfasst das nach dem erfindungsgemäßen Verfahren hergestellte Polymerisat P die Polymere A und B in einem Gewichtsverhältnis A:B im Bereich von 50:50 bis 98:2.

In einer Ausführungsform der Erfindung umfasst das nach dem erfindungsgemäßen Verfahren hergestellte Polymerisat P die Polymere A und B in einem Gewichtsverhältnis A:B im Bereich von 2:1 bis 20:1.

In einer Ausführungsform der Erfindung umfasst das nach dem erfindungsgemäßen Verfahren hergestellte Polymerisat P die Polymere A und B in einem Gewichtsverhältnis A:B im Bereich von 5:1 bis 10:1.

### Polymerisation

Die Polymerisate P werden hergestellt, indem eines der Polymere A oder B in Gegenwart des jeweils anderen Polymers hergestellt wird. Die Polymerisation beider Polymere erfolgt vorzugsweise in einem System.

Es gibt erfindungsgemäß mehrere Möglichkeiten:
Bei Verfahren 1 wird die Herstellung des zweiten Polymers, d.h. des jeweils anderen Polymers, erst gestartet, wenn die Herstellung des ersten Polymers nahezu vollständig abgeschlossen ist.

Bei Verfahren 1 ("Nacheinander") wird also beispielsweise die Polymerisation der Monomere b1) und b2) erst gestartet, wenn die Polymerisation der Monomere a1) bis a4) nahezu vollständig abgeschlossen ist.

Bei Verfahren 2 ("Parallel 1") wird die Herstellung des zweiten Polymers, d.h. des jeweils anderen Polymers, erst gestartet, wenn wenigstens 50 Gew.-% aller Monomere, die zur Herstellung des ersten Polymers verwendet werden, polymerisiert sind.

Bei Verfahren 2 wird also beispielsweise die Polymerisation der Monomere b1) und b2) gestartet, wenn mindestens 50 Gew.-% der Gesamtmenge der Monomere a1) bis a4) polymerisiert sind.

Bei Verfahren 3 ("Parallel 2") wird die Herstellung des zweiten Polymers, d.h. des jeweils anderen Polymers erst gestartet, wenn im Bereich von 10 bis 50 Gew.-% aller Monomere, die zur Herstellung des ersten Polymers, d. h. Polymer A oder Polymer B, insgesamt verwendet werden, polymerisiert sind und beendet, bevor 80 Gew.-% aller Monomere, die zur Herstellung des ersten Polymers verwendet werden, polymerisiert sind.

Bei Verfahren 3 wird also beispielsweise die Polymerisation der Monomere b1) und b2) gestartet, wenn im Bereich von 10 bis 50 Gew.-% der Gesamtmenge aller Monomere a1) bis a4) polymerisiert sind und beendet, bevor 80 Gew.-% der Gesamtmenge aller Monomere a1) bis a4) polymerisiert sind.

Die Polymerisation der Monomere zur Bildung des Gemischs aus den Polymeren A und B erfolgt in der Regel in einem System, ohne die Notwendigkeit, eines der Polymere A oder B vorab separat zu polymerisieren. Eine Zweistufenpolymerisation als zweistufiges Zulaufverfahren ist bevorzugt.

### Verfahren 1: "Nacheinander"

Eine Ausführungsform des erfindungsgemäßen Verfahrens ist dadurch gekennzeichnet, dass die Monomere des jeweils anderen Polymers erst dann hinzugegeben werden, wenn mehr als 99 Gew.-% bevorzugt mehr als 99,9 Gew.-% , insbesondere mehr als 99,99 Gew.-% der Menge aller zur Polymerisation von Polymer A oder Polymer B einzusetzenden Monomere polymerisiert sind.

In einer Ausführungsform der Erfindung beginnt die Herstellung von Polymer B, also die Polymerisation der Monomere b1) und b2) erst dann, wenn mehr als 99 Gew.-%, bevorzugt mehr als 99,9 Gew.-%, insbesondere mehr als 99,99 Gew.-% der Menge aller zur Herstellung des Polymers A einzusetzenden Monomere a1) bis a4) polymerisiert sind.

In einer weiteren Ausführungsform der Erfindung beginnt die Herstellung von Polymer A, also die Polymerisation der Monomere a1) bis a4) erst dann, wenn mehr als 99 Gew.-%, bevorzugt mehr als 99,9 Gew.-%, insbesondere mehr als 99,99 Gew.-% der Menge aller zur Herstellung des Polymers B einzusetzenden Monomere b1) und b2) polymerisiert sind.

Bei diesem bevorzugten erfindungsgemäßen Verfahren findet eine Copolymerisation der Monomere a1) bis a4) mit den Monomeren b1) und b2) nur in untergeordnetem Ausmaß statt.

### Verfahren 2: "Parallel 1"

Eine weitere Ausführungsform des erfindungsgemäßen Verfahrens ist dadurch gekennzeichnet, dass die Herstellung des jeweils anderen Polymers, also die Zugabe der Monomere a1) bis a4) bzw. b1) und b2) dann gestartet wird, wenn im Bereich von 50 bis 99 Gew.-% aller zur Polymerisation von Polymer A oder Polymer B einzusetzenden Monomere polymerisiert sind.

In einer Ausführungsform der Erfindung wird die Herstellung von Polymer A, also die Zugabe der Monomere a1) bis a4) gestartet, wenn im Bereich von 50 bis 99 Gew.-% aller zur Herstellung von Polymer B einzusetzenden Monomere b1) und b2) polymerisiert sind.

In einer Ausführungsform der Erfindung wird die Herstellung von Polymer B, also die Zugabe der Monomere b1) und b2) gestartet, wenn im Bereich von 50 bis 99 Gew.-% aller zur Herstellung von Polymer A einzusetzenden Monomere a1) bis a4) polymerisiert sind.

Je nachdem, wann die Polymerisation des zweiten Polymers A oder B gestartet wird, findet bei diesem erfindungsgemäßen Verfahren eine Copolymerisation der Monomere a1) bis a4) mit b1) und b2) in mehr oder weniger deutlichem Ausmaß statt.

### Verfahren 3: "Parallel 2"

Eine weitere Ausführungsform des erfindungsgemäßen Verfahrens ist dadurch gekennzeichnet, dass die Herstellung des jeweils anderen Polymers, also die Zugabe der Monomere a1) bis a4) oder b1) und b2) erst gestartet wird, wenn im Bereich von 10 bis 50 Gew.-% aller zur Polymerisation von Polymer A oder Polymer B einzusetzenden Monomere polymerisiert sind und beendet wird, bevor mehr als 80 Gew.-% der Menge aller zur Polymerisation von Polymer A oder Polymer B einzusetzenden Monomere polymerisiert sind.

In einer Ausführungsform der Erfindung wird die Herstellung von Polymer A, also die Zugabe der Monomere a1) bis a4) erst gestartet, wenn im Bereich von 10 bis 50 Gew.-% aller zur Herstellung von Polymer B einzusetzenden Monomere b1) und b2) polymerisiert sind und beendet, bevor mehr als 80 Gew.-% der Menge aller zur Herstellung von Polymer B einzusetzenden Monomere b1) und b2) polymerisiert sind.

In einer Ausführungsform der Erfindung wird die Herstellung von Polymer B, also die Zugabe der Monomere b1) und b2) erst gestartet, wenn im Bereich von 10 bis 50 Gew.-% aller zur Herstellung von Polymer A einzusetzenden Monomere a1) bis a4) polymerisiert sind und beendet, bevor mehr als 80 Gew.-% der Menge aller zur Herstellung von Polymer A einzusetzenden Monomere a1) bis a4) polymerisiert sind.

Eine bevorzugte Ausführungsform der Erfindung ist ein Verfahren, bei dem die Herstellung bzw. Polymerisation von Polymer B, also die Zugabe der Monomere b1) und b2) gestartet wird, wenn mehr als 99 Gew.-%, bevorzugt mehr als 99,9 Gew.-% , insbesondere mehr als 99,99 Gew.-% der Menge aller zur Herstellung des Polymers A einzusetzenden Monomere a1) bis a4) polymerisiert sind.

Eine bevorzugte Ausführungsform der Erfindung ist also ein Verfahren zur Herstellung von Polymerisaten P umfassend ein Gemisch aus wenigstens einem Polymer A und wenigstens einem Polymer B, wobei
A) Polymer A in einpolymerisierter Form umfasst
   a1) 30 bis 70 Gew.-% C₁-C₄-Alkyl-(Meth)acrylat,
   a2) 30 bis 70 Gew.-% (Meth)Acrylsäure,
   a3) 0 bis 20 Gew.-% C₈-C₃₀-Alkylsubstituierte Monomere,
   a4) 0 bis 2 0 Gew.-% weitere von a1) bis a2) verschiedene Monomere, wobei sich die Mengen von a1) bis a4) zu 100 Gew.-% addieren, und
B) Polymer B in einpolymerisierter Form umfasst
   b1) wenigstens ein Monomer b1), das wenigstens eine Amidgruppe trägt, und
   b2) gegebenenfalls weitere von b1) verschiedene Monomere b1), das dadurch gekennzeichnet ist, dass die Polymerisation bzw. Zugabe der Monomere b1) und b2) erst dann gestartet wird, wenn mehr als 99 Gew.-% der Menge aller zur Herstellung von Polymer A einzusetzenden Monomere a1) bis a4) polymerisiert sind.

Eine bevorzugte Ausführungsform der Erfindung ist ein Verfahren zur Herstellung von Polymerisaten P umfassend ein Gemisch aus wenigstens einem Polymer A und wenigstens einem Polymer B, wobei
A) Polymer A in einpolymerisierter Form umfasst
   a1) 40 bis 60 Gew.-% C₁-C₄-Alkyl-(Meth)acrylat,
   a2) 35 bis 59 Gew.-% (Meth)Acrylsäure,
   a3) 0,1 bis 10 Gew.-% C₈-C₃₀-Alkylsubstituierte Monomere,
   a4) 0 bis 3 Gew.-% weitere von a1) bis a2) verschiedene Monomere, wobei sich die Mengen von a1) bis a4) zu 100 Gew.-% addieren, und
B) Polymer B in einpolymerisierter Form umfasst
   b1) wenigstens ein Monomer b1), das wenigstens eine Amidgruppe trägt, und
   b2) gegebenenfalls weitere von b1) verschiedene Monomere b1), das dadurch gekennzeichnet ist, dass die Polymerisation bzw. Zugabe der Monomere b1) und b2) erst dann gestartet wird, wenn mehr als 99 Gew.-% der Menge aller zur Herstellung von Polymer A einzusetzenden Monomere a1) bis a4) polymerisiert sind.

Eine weitere bevorzugte Ausführungsform der Erfindung ist ein Verfahren zur Herstellung von Polymerisaten P umfassend ein Gemisch aus wenigstens einem Polymer A und wenigstens einem Polymer B, wobei
A) Polymer A in einpolymerisierter Form umfasst
   a1) 40 bis 60 Gew.-% Ethylacrylat,
   a2) 35 bis 59 Gew.-% Methacrylsäure,
   a3) 0,1 bis 10 Gew.-% Monomeren der allgemeinen Formel a3.1) 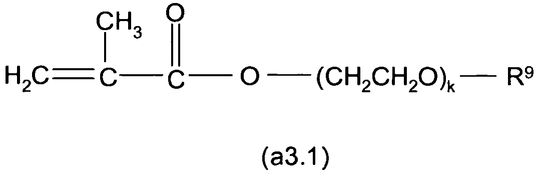 wobei
      k eine ganze Zahl im Bereich von 10 bis 30 ist und
      R⁹ linearer C₁₂-C₁₈-Alkylrest ist,
   a4) 0 bis 3 Gew.-% weitere von A)a1) bis A)a2) verschiedene Monomere, wobei sich die Mengen von A)a1) bis A)a4) zu 100 Gew.-% addieren, und
C) Polymer B in einpolymerisierter Form umfasst
   b1) Methacrylamid und
   b2) gegebenenfalls weitere von b1) verschiedene Monomere b2), das dadurch gekennzeichnet ist, dass die Polymerisation bzw. Zugabe der Monomere b1) und b2) erst dann gestartet wird, wenn mehr als 99 Gew.-% der Menge aller zur Herstellung von Polymer A einzusetzenden Monomere a1) bis a4) polymerisiert sind.

Bevorzugt ist das erfindungsgemäße Verfahren eine radikalische Polymerisation. Das erfindungsgemäße Verfahren kann beispielsweise als Lösungspolymerisation, Fällungspolymerisation oder Emulsionspolymerisation durchgeführt werden. Die Emulsionspolymerisation ist beispielsweise beschrieben in H. Rauch-Puntigam, Th.Völker, Acryl- und Methacrylverbindungen, Springer Verlag, Berlin, 1967**.**

Bevorzugt ist das erfindungsgemäße Verfahren eine wässrige radikalische Emulsionspolymerisation. Diese wird in Gegenwart von ionischen oder nichtionischen Emulgatoren zur Emulgierung und zur Stabilisierung des entstehenden Polymerisats B durchgeführt.

### Radikalstarter

Als Radikalstarter (Initiatoren) können die dem Fachmann bekannten Substanzen verwendet werden. Geeignete Radikalstarter sind beispielsweise beschrieben in WO 2007/017434, S.31, Z.28 bis S.32, Z.22, worauf hiermit Bezug genommen wird. Die Radikalstarter werden bevorzugt eingesetzt in Mengen im Bereich von 0,02 bis 2 Gew.-%, bezogen auf die Gesamtmenge der polymerisierbaren Monomere a1) bis a4) und b1) bis b2) von 100 Gew.-%. Das erfindungsgemäße Verfahren wird bevorzugt bei Temperaturen von 30 bis 100°C durchgeführt.

### Molekulargewichtsregler

In einer Ausführungsform der Erfindung werden zur Regulierung des Molekulargewichts der Polymerisate sogenannte Molekulargewichtsregler verwendet. Molekulargewichtsregler sind Verbindungen mit radikalübertragenden Eigenschaften. Bevorzugte Molekulargewichtsregler sind Mercaptane wie z.B. 2-Ethylhexylthioglycolat, n-Butylmercaptan, n- bzw. t-Dodecylmercaptan oder Pentaerythrittetrathioglycolat.

Weitere geeignete Molekulargewichtsregler sind beschrieben in WO 2007/017434, S.32, Z.27 bis S.33, Z.21, worauf hiermit Bezug genommen wird.

### Neutralisation

Die Säuregruppen der erfindungsgemäß hergestellten Polymerisate können protoniert, d.h. nichtionisch oder teilweise oder vollständig deprotoniert, d.h. ionisch vorliegen. Insbesondere zur Verwendung der Polymere in haarkosmetischen Zubereitungen ist eine teilweise oder vollständige Neutralisation der Säuregruppen vorteilhaft.

Die wenigstens teilweise Neutralisation der Säuregruppen erfindungsgemäß hergestellten Polymerisate P kann durch Umsetzung mit dem Fachmann bekannten Basen wie Alkalihydroxiden, Ammoniak oder Aminen erfolgen.

Erfindungsgemäß geeignete Neutralisationsmittel sind in WO 2007/024457, S.17, Z.4 bis Z.40 beschrieben, worauf hiermit Bezug genommen wird.

Bevorzugte Neutralisationsmittel sind 2-Amino-2-methylpropanol, Triethanolamin, 2-Amino-2-ethylpropan-1,3-diol, N,N-Dimethylaminoethanol oder 3-Diethylamino-1-propylamin.

### Vernetzer

In einer Ausführungsform der Erfindung werden zur Herstellung der Polymerisate B nach dem erfindungsgemäßen Verfahren sogenannte Vernetzer verwendet. Geeignete Vernetzer sind beschrieben in WO 2007/024457, S.7, Z.1 bis S.9, Z.2, worauf hiermit Bezug genommen wird.

Erfindungsgemäß sind auch die Polymerisate P erhältlich nach dem erfindungsgemäßen Verfahren.

Erfindungsgemäß ist auch die Verwendung eines erfindungsgemäßen Polymerisates P als Verdickungsmittel, bevorzugt als Verdickungsmittel für kosmetische Zubereitungen, insbesondere als Verdickungsmittel für haarkosmetische Zubereitungen.

Erfindungsgemäß ist auch ein Verfahren zur Behandlung von Haaren dadurch gekennzeichnet, dass die Haare mit einem erfindungsgemäßen Polymerisat P in Kontakt gebracht werden.

Außer im bevorzugten Bereich der kosmetischen Zubereitungen eignen sich die erfindungsgemäßen Polymerisate aufgrund ihrer Verdickungswirkung in wässrigen Systemen und ihren filmbildenden Eigenschaften zur Verwendung als Kleb-, Bindemittel und Festiger in zahlreichen weiteren Anwendungsgebieten.

So sind die erfindungsgemäßen Polymerisate als pharmazeutische Hilfsmittel wie Tablettenbindemittel, Verdicker, Elektrodengele oder Hautklebegele und Waschmitteladditive wie Verdicker, Soil Release sowie als Hilfsmittel für zahlreiche technische Anwendungen wie Klebstoffe (Verdicker für Klebestifte), Papier (Papierhilfsmittel, Verdicker für Papierstreichfarben), Pigmentdispersionen, Textilhilfsmittel, Ionenaustauscher, tertiäre Erdölgewinnung und insbesondere auch Pflanzenschutzzubreitungen geeignet.

Erfindungsgemäß ist es auch, die erfindungsgemäßen Polymerisate P mittels bekannter Verfahren, beispielsweise durch Sprühtrocknung der durch Emulsionspolymerisation hergestellten Polymerisate P, als Feststoffe zu isolieren.

Die derart gewonnenen Polymerpulver sind einfach zu transportieren und zu lagern und können in entsprechende Zubereitungen eingearbeitet werden.

### Kosmetische Zubereitungen

Erfindungsgemäß sind auch kosmetische Zubereitungen, bevorzugt haarkosmetische Zubereitungen enthaltend ein Polymerisat P erhältlich nach dem erfindungsgemäßen Verfahren.

Die Erfindung betrifft weiterhin kosmetische Zubereitungen, die die durch das erfindungsgemäße Verfahren erhältlichen Polymerisate P umfassen.

Solche erfindungsgemäßen kosmetische Zubereitungen sind beispielsweise ausgewählt aus Gelcremes, Hydroformulierungen, Stiftformulierungen, kosmetischen Ölen und Ölgelen, Mascara, Selbstbräunem, Gesichtspflegemitteln, Körperpflegemitteln, After-Sun-Präparaten, Haarverformungsmitteln und Haarfestigern.

Weitere erfindungsgemäße kosmetische Zubereitungen sind hautkosmetische Zubereitungen, insbesondere solche zur Pflege der Haut. Diese liegen insbesondere als W/O- oder O/W-Hautcremes, Tag- und Nachtcremes, Augencremes, Gesichtscremes, Antifaltencremes, Mimikcremes, Feuchthaltecremes, Bleichcremes, Vitamincremes, Hautlotionen, Pflegelotionen und Feuchthaltelotionen vor.

Weiterhin eignen sich die Polymerisate P als Inhaltsstoffe für hautkosmetische Zubereitungen wie Gesichtswässer, Gesichtsmasken, Deodorantien und andere kosmetische Lotionen und für die Verwendung in der dekorativen Kosmetik, beispielsweise als Abdeckstift, Theaterfarbe, in Mascara und Lidschatten, Lippenstiften, Kajalstiften, Eyelinern, Makeup, Grundierungen, Rouges und Pudern und Augenbrauenstiften.

Außerdem können die Polymerisate P verwendet werden in Nose-Strips zur Porenreinigung, in Antiaknemitteln, Repellentien, Rasiermitteln, Haarentfernungsmitteln, Intimpflegemitteln, Fußpflegemitteln sowie in der Babypflege.

Weitere bevorzugte erfindungsgemäße Zubereitungen sind Wasch-, Dusch- und Badepräparate, die die Polymerisate P enthalten.

Unter Wasch-, Dusch- und Badepräparaten werden im Rahmen dieser Erfindung Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes verstanden.

Die erfindungsgemäßen kosmetischen Zubereitungen können als wässrige oder wässrigalkoholische Lösungen, O/W- sowie W/O-Emulsionen, Hydrodispersionsformulierungen, feststoffstablilisierte Formulierungen, Stiftformulierungen, PIT-Formulierungen, in Form von Cremes, Schäumen, Sprays (Pumpspray oder Aerosol), Gelen, Gelsprays, Lotionen, Ölen, Ölgelen oder Mousse vorliegen und dementsprechend mit üblichen weiteren Hilfsstoffen formuliert werden.

Die erfindungsgemäßen kosmetischen Zubereitungen enthalten bevorzugt wenigstens ein Polymerisat P, wenigstens einen kosmetisch akzeptablen Träger und wenigstens einen davon verschiedenen Bestandteil der ausgewählt ist unter kosmetisch aktiven Wirkstoffen, Emulgatoren, Tensiden, Konservierungsmitteln, Parfümölen, weiteren Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnem, Pflegemitteln, Färbemitteln, Tönungsmitteln, Bräunungsmitteln, Farbstoffen, Pigmenten, Konsistenzgebern, Feuchthaltemitteln, Rückfettem, Collagen, Eiweisshydrolysaten, Lipiden, Antioxidantien, Entschäumern, Antistatika, Emollienzien und Weichmachern.

Erfindungsgemäß bevorzugte Haarpflegemittel sind ausgewählt aus Vorbehandlungsmitteln, Haarspülungen, Haarconditionern, Haarbalsamen, leave-on-Haarkuren, rinse-off-Haarkuren, Haarwässern, Pomaden, Frisiercremes, Frisierlotionen, Frisiergelen, Spitzenfluids, Hot-Oil-Treatments und Schaumkuren.

Bevorzugt werden die Polymerisate P als rheologiemodifizierende Filmbildner, Haarfestiger und Konditioniermittel zur Herstellung von kosmetischen, bevorzugt haarkosmetischen Zubereitungen verwendet.

Ein weiterer Gegenstand der Erfindung sind also kosmetische, insbesondere haarkosmetische Zubereitungen enthaltend wenigstens ein Polymerisat P.

Bevorzugte haarkosmetische Zubereitungen sind Haarreinigungsmittel, Shampoos, Haarpflegemittel, Haarfärbezubereitungen und Haarfestiger, darunter insbesondere Haarfestigergele.

Die Polymerisate P wirken insbesondere als filmbildende und/oder konditionierende Rheologiemodifizierungsmittel. Sie eignen sich somit speziell für Haarfestiger als "verdickende Festiger" oder "festigende Verdicker" und in Haarpflegemitteln als "konditionierende Verdicker".

Prinzipiell können die Polymerisate P bei ihrer Verwendung in mehrphasigen Zubereitungen wie beispielsweise O/W und W/O, sowohl in der Wasserphase als auch in der Ölphase eingesetzt werden. Im Allgemeinen enthalten heterogenphasige flüssig/flüssig-Zubereitungen die Polymerisate P im Wesentlichen in der Wasserphase.

Ein weiterer Gegenstand der Erfindung sind haarkosmetische Mittel enthaltend
A) wenigstens ein Polymerisat P,
B) gegebenenfalls wenigstens ein von A) verschiedenes Haarpolymer,
C) wenigstens einen kosmetisch akzeptablen Träger, und
D) gegebenenfalls wenigstens einen von A) und B) verschiedenen kosmetisch akzeptablen Wirk- und/oder Hilfsstoff.

Die Polymerisate P in den haarkosmetischen Mitteln auch als haarfestigende Komponente eingesetzt werden, so dass der Einsatz weiterer Festigerpolymere nur in verringerter Menge erforderlich ist oder sogar ganz überflüssig sein kann.

Die Polymerisate P zeichnen sich vorteilhafterweise auch durch konditionierende Eigenschaften aus und können die sensorischen Eigenschaften des Haars verbessern, z. B. ihm Geschmeidigkeit und Glanz verleihen.

Die haarkosmetischen Mittel enthalten die Polymerisate P vorzugsweise in einem Anteil von etwa 0,1 bis 10 Gew.-%, besonders bevorzugt 0,2 bis 6 Gew.-%, insbesondere 0,3 bis 3 Gew.-%, bezogen auf das Gesamtgewicht des Mittels.

Beispiele für geeignete Haarpolymere B) und deren bevorzugte Mengen sind in WO 2007/010035, S.68, Z.32 bis S.70, Z.22 ausführlich beschrieben. Auf diese Textstelle wird hiermit in vollem Umfang Bezug genommen.

Vorzugsweise weisen die Zubereitungen eine Trägerkomponente C) auf, die ausgewählt ist unter Wasser, hydrophilen Komponenten, hydrophoben Komponenten und Mischungen davon.

Geeignete Trägerkomponenten C) sind in WO 2007/010035, S.70, Z.28 bis S.71, Z.37 ausführlich beschrieben. Auf diese Textstelle wird hiermit in vollem Umfang Bezug genommen.

Zusätzlich können die erfindungsgemäßen Mittel als Komponente D) wenigstens einen weiteren, von A) und B) verschiedenen kosmetischen Wirk- oder Hilfsstoff enthalten. Geeignete Komponenten D) sind in WO 2007/010035, S.72, Z.2 bis S.72, Z.13 ausführlich beschrieben. Auf diese Textstelle wird hiermit in vollem Umfang Bezug genommen.

Die Polymerisate P können gemeinsam mit bekannten Verdickern verwendet werden. Geeignete Verdicker sind in WO 2007/010035, S.72, Z.15 bis S.72, Z.24 ausführlich beschrieben. Auf diese Textstelle wird hiermit in vollem Umfang Bezug genommen.

### Konditionierungsmittel

Als Konditionierungsmittel für die erfindungsgemäßen kosmetischen Zubereitungen werden bevorzugt diejenigen Konditionierungsmittel gewählt, die auf Seite 34, Zeile 24 bis Seite 37, Zeile 10 der WO 2006/106140 beschrieben sind, worauf hiermit Bezug genommen wird.

### Verdicker

Für Gele, Shampoos und Haarpflegemittel geeignete Verdicker sind in "Kosmetik und Hygiene von Kopf bis Fuß", Hrsg. W. Umbach, 3. Auflage, Wiley-VCH, 2004, S.235-236 genannt, worauf an dieser Stelle vollumfänglich Bezug genommen wird.

Geeignete weitere Verdickungsmittel für die erfindungsgemäßen kosmetischen Zubereitungen sind beispielsweise auch auf Seite 37, Zeile 12 bis Seite 38, Zeile 8 der WO 2006/106140 beschrieben, worauf hiermit Bezug genommen wird.

### Konservierungsmittel

Geeignete Konservierungsmittel für die erfindungsgemäßen kosmetischen Zubereitungen sind beispielsweise auf Seite 38, Zeile 10 bis Seite 39, Zeile 18 der WO 2006/106140 beschrieben, worauf hiermit Bezug genommen wird.

### UV-Lichtschutzfilter

Geeignete UV- Lichtschutzfilter für die erfindungsgemäßen kosmetischen Zubereitungen sind beispielsweise auf Seite 39, Zeile 20 bis Seite 41 Zeile 10 der WO 2006/106140 beschrieben, worauf hiermit Bezug genommen wird.

### Antioxidantien

Geeignete Antioxidantien für die erfindungsgemäßen kosmetischen Zubereitungen sind beispielsweise auf Seite 41, Zeile 12 bis Seite 42 Zeile 33 der WO 2006/106140 beschrieben, worauf hiermit Bezug genommen wird.

### Dispergiermittel

Wenn in den erfindungsgemäßen Zubereitungen unlösliche Wirkstoffe, z.B. Antischuppenwirkstoffe oder Siliconöle, dispergiert und auf Dauer in Schwebe gehalten werden sollen, werden bevorzugt Dispergiermittel und Verdicker wie z. B. Magnesium-Aluminium-Silicate, Bentonite, Fettacyl-Derivate, Polyvinylpyrrolidon oder Hydrokolloide, z. B. Xanthan Gum oder Carbomere, eingesetzt.

Die Zubereitungen können weitere in der Kosmetik übliche Zusatzstoffe, beispielsweise Parfüm, Farbstoffe, rückfettende Agentien, Komplexierungs- und Sequestrierungsagentien, Perlglanzagentien, Pflanzenextrakte, Vitamine, Wirkstoffe, Pigmente, die eine färbende Wirkung haben, weichmachende, anfeuchtende und/oder feuchthaltende Substanzen, oder andere übliche Bestandteile wie Alkohole, Polyole, Polymere, organische Säuren zur pH-Wert-Einstellung, Schaumstabilisatoren, Elektrolyte, organische Lösemittel oder Silikonderivate enthalten.

Hinsichtlich der genannten, dem Fachmann bekannten weiteren Inhaltsstoffe für die Zubereitungen sei auf "Kosmetik und Hygiene von Kopf bis Fuß", Hrsg. W. Umbach, 3. Auflage, Wiley-VCH, 2004, S.123-128 verwiesen, worauf hiermit Bezug genommen wird.

Die erfindungsgemäßen Zubereitungen wie Haarsprays, Gele, Shampoos und Haarpflegemittel enthalten gegebenenfalls ethoxylierte Öle ausgewählt aus der Gruppe der ethoxylierten Glycerin-Fettsäureester, insbesondere bevorzugt PEG-10 Olivenölglyceride, PEG-11 Avocadoölglyceride, PEG-11 Kakaobutterglyceride, PEG-13 Sonnenblumenölglyceride, PEG-15 Glycerylisostearat, PEG-9 Kokosfettsäureglyceride, PEG-54 Hydriertes Ricinusöl, PEG-7 Hydriertes Ricinusöl, PEG-60 Hydriertes Ricinusöl, Jojobaöl Ethoxylat (PEG-26 Jojoba-Fett-Säuren, PEG-26 Jojobaalkohol), Glycereth-5 Cocoat, PEG-9 Kokosfettsäureglyceride, PEG-7 Glycerylcocoat, PEG-45 Palmkernölglyceride, PEG-35 Ricinusöl, Olivenöl-PEG-7 Ester, PEG-6 Caprylisäure/Caprinsäureglyceride, PEG-10 Olivenölglyceride, PEG-13 Sonnenblumenölglyceride, PEG-7 Hydriertes Ricinusöl, Hydrierte Palmkemölglycerid-PEG-6 Ester, PEG-20 Maisölglyceride, PEG-18 Glycerylolead-cocoat, PEG-40 Hydriertes Ricinusöl, PEG-40 Ricinusöl, PEG-60 Hydriertes Ricinusöl, PEG-60 Maisölglyceride, PEG-54 Hydriertes Ricinusöl, PEG-45 Palmkernölglyceride, PEG-80 Glycerylcocoat, PEG-60 Mandelölglyceride, PEG-60 "Evening Primrose" Glyceride, PEG-200 Hydriertes Glycerylpalmat, PEG-90 Glycerylisostearat.

Bevorzugte ethoxylierte Öle sind PEG-7 Glycerylcocoat, PEG-9 Kokosglyceride, PEG-40 Hydriertes Rizinusöl, PEG-200 hydriertes Glycerylpalmat.

Ethoxylierte Glycerin-Fettsäureester werden in wässrigen Reinigungsrezepturen zu verschiedenen Zwecken eingesetzt. Glycerin-Fettsäureester mit einem Ethoxylierungsgrad von ca. 30-50 dienen als Lösungsvermittler für unpolare Substanzen wie Parfumöle. Hochethoxylierte Glycerin-Fettsäureester werden als Verdicker eingesetzt.

### Wirkstoffe

Vorteilhafte Wirkstoffe für die erfindungsgemäßen kosmetischen Zubereitungen sind beispielsweise auf Seite 44, Zeile 24 bis Seite 49 Zeile 39 der WO 2006/106140 beschrieben, worauf hiermit Bezug genommen wird.

### UV-Lichtschutzmittel

Die erfindungsgemäßen Zubereitungen enthalten in einer bevorzugten Ausführungsform UV-Lichtschutzmittel zum Schutz der Haut und/oder der Haare. Geeignete UV-Lichtschutzmittel sind in der WO 2006/106114, S.24, Z.4 bis S.27, Z.27 ausführlich beschrieben, worauf hiermit in vollem Umfang Bezug genommen wird.,

### Perlglanzwachse

Geeignete Perlglanzwachse für die erfindungsgemäßen kosmetischen Zubereitungen sind beispielsweise auf Seite 50, Zeile 1 bis Zeile 16 der WO 2006/106140 beschrieben, worauf hiermit Bezug genommen wird.

### Emulgatoren

Die erfindungsgemäßen kosmetischen Zubereitungen liegen in einer bevorzugten Ausführungsform der Erfindung in Form von Emulsionen vor. Die Herstellung solcher Emulsionen erfolgt nach bekannten Methoden. Geeignete Emulgatoren für die erfindungsgemäßen Emulsionen sind beispielsweise auf Seite 50, Zeile 18 bis Seite 53, Zeile 4 der WO 2006/106140 beschrieben, worauf hiermit Bezug genommen wird.

### Parfümöle

Sollen den erfindungsgemäßen kosmetischen Zubereitungen Parfumöle zugesetzt werden, so sind geeignete Parfümöle beispielsweise auf Seite 53, Zeile 10 bis Seite 54, Zeile 3 der WO 2006/106140 beschrieben, worauf hiermit Bezug genommen wird.

### Pigmente

Gegebenenfalls enthalten die erfindungsgemäßen kosmetischen Zubereitungen weiterhin Pigmente. Geeignete Pigmente für die erfindungsgemäßen Zubereitungen sind beispielsweise auf Seite 54, Zeile 5 bis Seite 55, Zeile 19 der WO 2006/106140 beschrieben, worauf hiermit Bezug genommen wird.

### Nanopartikel

Gegebenenfalls enthalten die erfindungsgemäßen Zubereitungen wasserunlösliche Nanopartikel, also Teilchen mit einer Teilchengröße im Bereich von 1 bis 200, bevorzugt von 5 bis 100 nm. Bevorzugte Nanopartikel sind Nanopartikel von Metalloxiden, insbesondere von Zinkoxid und/oder Titandioxid.

### Polymere

Die erfindungsgemäßen kosmetischen Zubereitungen enthalten in einer bevorzugten Ausführungsform außer den Polymerisaten P noch weitere Polymere. Geeignete weitere Polymere sind beispielsweise auf Seite 55, Zeile 21 bis Seite 63, Zeile 2 der WO 2006/106140 beschrieben. Auf den Inhalt der genannten Textstelle wird hiermit in vollem Umfang Bezug genommen.

Die Polymerisate P sind auch als rheologiemodifizierende Filmbildner in Haargelen, insbesondere sogenannten Stylinggelen geeignet.

Eine bevorzugte Ausführungsform der Erfindung sind haarkosmetische Zubereitungen, insbesondere Haarfestiger und Haargele, die neben den erfindungsgemäß erhältlichen Polymeren in der Kosmetik übliche Gelbildner enthalten.

Solche weiteren, üblichen Gelbildner sind leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycole Dicaprate Dicaprylate (and) PPG-1 Trideceth-6, Polyquaternium-7, Polyquaternium-44.

### Haarwaschmittel

Eine bevorzugte Ausführungsform der Erfindung sind Haarwaschmittel und Shampoos enthaltend die Polymerisate P.

An Shampoos und Haarwaschmittel werden je nach Haarqualität oder Kopfhautproblem gegebenenfalls zusätzliche Anforderungen gestellt.

Bevorzugte erfindungsgemäße Shampoos und Haarwaschmittel enthalten anionische Tenside. Weitere bevorzugte erfindungsgemäße Shampoos und Haarwaschmittel enthalten Kombinationen von anionischen und ampholytischen Tensiden. Weitere bevorzugte erfindungsgemäße Shampoos und Haarwaschmittel enthalten Kombinationen von anionischen und zwitterionischen Tensiden. Weitere bevorzugte erfindungsgemäße Shampoos und kosmetische Reinigungsmittel enthalten Kombinationen von anionischen und nichtionischen Tensiden.

Geeignete Tenside aller Typen sind zuvor unter "Tenside" bereits beschrieben.

Bevorzugte anionische Tenside sind Alkylsulfate, Alkylpolyglykolethersulfate und Ethercarbonsäuresalze mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen. Besonders bevorzugte anionische Tenside sind die Alkali- oder Ammoniumsalze des Laurylethersulfates mit einem Ethoxylierungsgrad von 2 bis 4 EO-Einheiten.

Ein bevorzugtes zwitterionisches Tensid ist das unter der INCI-Bezeichnung Cocamidopropyl Betaine bekannte Fettsäureamid-Derivat.

Besonders bevorzugte ampholytische Tenside sind das N-Kokosalkylaminopropionat, das Kokosacylaminoethylaminopropionat und das C12-C12-Acylsarcosin. Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

### Darreichung

Die erfindungsgemäßen Zubereitungen können beispielsweise als aus Aerosolbehältem, Quetschflaschen oder durch eine Pump-, Sprüh- oder Schaumvorrichtung versprühbare Präparate vorliegen, jedoch auch in Form eines aus normalen Flaschen und Behältern auftragbaren Mittels. Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Dimethylether, Kohlenwasserstoffe (Propan, Butan, Isobutan) geeignet, die allein oder in Mischung, beispielsweise Mischungen aus Dimethylether und Isobutan oder Dimethylether und Butan, miteinander eingesetzt werden können. Auch Druckluft, Stickstoff, Stickstoffdioxid oder Kohlendioxid oder Gemische aus diesen Substanzen sind vorteilhaft zu verwenden.

Die Herstellung der erfindungsgemäßen Zubereitungen kann in der üblichen Weise durch Mischen der einzelnen Bestandteile erfolgen. Der pH-Wert der Zubereitungen kann in bekannter Weise durch Zugabe von Säuren oder Basen eingestellt werden, vorzugsweise durch Zugabe von Puffergemischen, z.B. auf Basis von Citronensäure/Citrat oder Phosphorsäure/Phosphat-Puffergemischen. In einer Ausführungsform der Erfindung liegt der pH-Wert unter 10, z.B. im Bereich von 2-7, insbesondere im Bereich von 3-5.

Bevorzugte Shampooformulierungen enthalten
a) 0,05 bis 10 Gew.-% wenigstens eines erfindungsgemäß erhältlichen Polymers,
b) 25 bis 94,95 Gew.-% Wasser,
c) 5 bis 50 Gew.-% Tenside,
d) 0 bis 5 Gew.-% eines Konditioniermittels,
e) 0 bis 10 Gew.-% weitere kosmetische Bestandteile.

In einer weiteren Ausführungsform können durch den Einsatz der Polymerisate P auch tensidreduzierte Formulierungen mit weniger als 10 Gew.-% Tensid, bezogen auf die Zubereitung, in einer für die Zubereitung ausreichenden Viskosität hergestellt werden.

In den Shampoos und kosmetischen Reinigungsmitteln können alle in Shampoos und kosmetischen Reinigungsmitteln üblicherweise eingesetzten anionischen, neutralen, amphoteren oder kationischen Tenside verwendet werden. Geeignete Tenside wurden vorstehend genannt. Besonders bevorzugt sind Shampoos und kosmetische Reinigungsmittel mit einem Tensidgehalt von mehr als 10 Gew.-%.

In den Shampooformulierungen können zur Erzielung bestimmter Effekte weitere Konditioniermittel eingesetzt werden. Hierzu zählen beispielsweise kationische Polymere mit der IN-CI-Bezeichnung Polyquaternium, insbesondere Copolymere aus Vinylpyrrolidon/N-Vinylimidazoliumsalzen (Luviquat®FC, Luviquat®HM, Luviquat®MS, Luviquat®Care), Copolymere aus N-Vinylpyrrolidon/Dimethylaminoethylmethacrylat, quatemisiert mit Diethylsulfat (Luviquat®P Q 11), Copolymere aus N-Vinylcaprolactam/N-Vinylpyrrolidon/N-Vinylimidazoliumsalzen (Luviquat®Hold); kationische Cellulosederivate (Polyquaternium-4 und -10), Acrylamidcopolymere (Polyquaternium-7).

Vorteilhafte Konditionierungsmittel stellen beispielsweise die nach INCl als Polyquaternium bezeichneten Verbindungen dar (insbesondere Polyquaternium-1 bis Polyquatemium-87).

### Beispiele

Die Erfindung wird nachfolgend durch Beispiele näher beschrieben ohne sie darauf zu beschränken. Sofern nicht anders angegeben, bedeuten Mengenangaben in "%" Gewichtsprozent.

### Durchführung des Biegetests

### Vorbereitung der Proben:

Das zu prüfende Polymer wurde in eine Gelformulierung eingearbeitet. 50g des hergestellten Gels wurden auf 220g mit entsalztem Wasser aufgefüllt und gelöst. Die gewogenen, trockenen Haarsträhnen (ca. 3g, 24cm Länge) wurden in die verdünnte Gelmasse getaucht. Durch 3maliges Eintauchen, Herausnehmen und Abstreifen wurde eine gleichmäßige Verteilung sichergestellt.

Die überschüssige Masse wurde zwischen Daumen und Zeigefinger abgestreift, die Haarsträhnen wurden anschließend durch Ausdrücken zwischen Filterpapier auf eine Gewichtszunahme von 1 - 1,4g (bezogen auf das Ausgangsgewicht der Haarsträhne, je nach Viskosität der Masse) eingestellt. Danach wurden die Strähnen von Hand so geformt, dass sie einen runden Querschnitt erhielten. Bei 20°C und 65% relativer Feuchte wurden die Strähnen über Nacht im Klimaraum getrocknet.

### Prüfung der Biegefestigkeit:

Die Prüfungen wurden in einem Klimaraum bei 20°C und 65% relativer Feuchte mittels eines Zug/Druck - Prüfgerätes (Typ Easytest 86 802, Fa. Frank) durchgeführt.

Die Haarsträhne wurde symmetrisch auf zwei zylindrische Rollen (Durchmesser 4 mm, Abstand = 90mm) der Probenaufnahme gelegt. Genau in der Mitte wurde nun von oben mit einem abgerundetem Stempel die Strähne ca. 40 mm bis zum Brechen des Gelfilmes durchgebogen. Die dafür erforderliche Kraft wurde mit einer Wägezelle (50 N) gemessen und in Newton angegeben.

### Bestimmung der Viskosität

Die Viskosität wurde mit einem Viskometer des Typs Brookfield DV-II+Pro bei 20°C gemessen. Für die Messung wurde Spindel Nr. 6 bei 20 Upm eingesetzt. Die Viskositätsbestimmung wurde an der neutralisierten Dispersion vorgenommen.

### Beispiel 1

Polymerisation von Methacrylamid in Gegenwart eines Copolymers aus Ethylacrylat, Methacrylsäure und einem Methacrylsäureester eines ethoxylierten C16 - C18-Fettalkoholgemisches -

Beginn der Polymerisation von Monomer b1) nachdem mehr als 99 Gew.-% der Monomere a1) bis a4) polymerisiert wurden

Der Versuchsaufbau bestand aus einem 2-Liter-Reaktionsgefäß mit Ankerrührer, Rückflußkühler und Zulaufgefäßen.

In der Vorlage wurden 260,0g VE-Wasser, 0,6g Natriumdodecylsulfat, 6.0g Methacrylsäure und 9,0g Ethylacrylat unter Rühren (120Upm) auf 80°C aufgeheizt.

Bei Erreichen von 80°C wurden 24,0g einer 2,5%igen wässrigen Natriumperoxodisulfatlösung für den Reaktionsstart schnell zugegeben.

5min nach der Zugabe der Natriumperoxodisulfatlösung wurde eine gerührte Emulsion bestehend aus:

| | |
|---|---|
| 140,0g | VE-Wasser |
| 1,65g | Natriumdodecylsulfat |
| 7,2g | Sorbitantristearat-20 EO (Tween®80, Fa.Croda) |
| 25.0g | Methacrylsäureester eines ethoxylierten C16 - C18-Fettalkoholgemisches (Visiomer®C18 PEG-1105-MA 60%ig, Fa. Evonik) |
| 109,0g | Methacrylsäure |
| 126,0g | Ethylacrylat |

während 1,5 Stunden in das Reaktionsgefäß zudosiert.

Nach Beendigung des Emulsionszulaufs wurde ein Zulauf aus 200,0g einer 15 Gew.-%igen wässrigen Methacrylamidlösung während 30min in das Reaktionsgefäß zudosiert.

Nach Zulaufende rührte der Versuch noch 2h bei 80°C nach.

Anschließend wurde der Versuch auf Raumtemperatur abgekühlt. Bei Erreichen von 40°C während des Abkühlprozesses erfolgte die Zugabe von 0,9g einer 1%igen wässrigen Wasserstoffperoxidlösung.

5min nach dieser Zugabe wurden 30,0g wässrige 0,25 Gew.-%ige L(+)-Ascorbinsäure während 30min zudosiert.

Die Dispersion wurde dann über einen 120µm Filter abfiltriert.

Der pH-Wert der 29,7 Gew.-%igen Dispersion lag bei ca. 2,9

Mit dieser Dispersion wurde ein Gel formuliert mit einem Anteil von 1,5 Gew.% der Dispersion (nur Feststoffanteil gerechnet)

| | |
|---|---|
| 10,1g | Dispersion aus Beispiel 1 |
| 166,0g | VE-Wasser |
| 20,0g | Luviskol®K90 (BASF SE) |
| 0,2g | ParFümöl "Lisa" (Drom Fragrances) |
| 0,8g | Cremophor®CO 40 (BASF SE) |
| 1,0g | Euxyl®PE 9010 (Schülke + Mayr) |

Diese Formulierung wurde unter Rühren mit Triethanolamin auf pH 7.0 eingestellt.

### Beispiel 2

Polymerisation von Methacrylamid in Gegenwart eines Copolymers aus Ethylacrylat, Methacrylsäure und einem Methacrylsäureester eines ethoxylierten C16 - C18-Fettalkoholgemisches - Beginn der Polymerisation von Monomer b1) nachdem im Bereich von 50-99 Gew.-% der Monomere a1) bis a4) polymerisiert sind.

Der Versuchsaufbau bestand aus einem 2-L-Reaktionsgefäß mit Ankerrührer, Rückflußkühler und Zulaufgefäßen.

In der Vorlage wurden 190,0g VE-Wasser, 0,6g Natriumdodecylsulfat, 6.0g Methacrylsäure und 9,0g Ethylacrylat unter Rühren (120Upm) auf 80°C aufgeheizt.

Bei Erreichen von 80°C wurden 24,0g einer 2,5 Gew.-%igen wässrigen Natriumperoxodisulfatlösung für den Reaktionsstart schnell zugegeben.

5min nach der Zugabe wurde eine gerührte Emulsion bestehend aus:

| | |
|---|---|
| 210,0g | VE-Wasser |
| 1,65g | Natriumdodecylsulfat |
| 7,2g | Sorbitantristearat-20 EO (Tween®80, Fa.Croda) |
| 25.0g | Methacrylsäureester eines ethoxylierten C16 - C18-Fettalkoholgemisches (Visiomer®C18 PEG-1105-MA 60%ig, Fa. Evonik) |
| 109,0g | Methacrylsäure |
| 126,0g | Ethylacrylat |

während 1h30min in das Reaktionsgefäß zudosiert.

1 h15min nach Start des Emulsionszulaufs wurde ein Zulauf aus 200,0g einer 15 Gew.-%igen wässrigen Methacrylamidlösung in 30min in das Reaktionsgefäß zugefahren.

Nach Zulaufende rührte der Versuch noch 2h bei 80°C nach.

Anschließend wurde der Versuch auf Raumtemperatur abgekühlt. Bei Erreichen von 40°C während des Abkühlprozesses erfolgte die Zugabe von 0,9g einer 1 gew.-%igen wässrigen Wasserstoffperoxidlösung.

5min nach dieser Zugabe wurden 30,0g wässrige 0,25 Gew.-%ige L(+)-Ascorbinsäure in 30min zudosiert.

Die Dispersion wurde dann über einen 120µm Filter abfiltriert.

Der pH-Wert der 29,7 Gew.-%igen Dispersion lag bei ca. 2,9

Mit dieser Dispersion wurde ein Gel formuliert mit einem Anteil von 1,5 Gew.% der Dispersion (nur Feststoffanteil gerechnet)

| | |
|---|---|
| 9,9g | Dispersion Beispiel 2 |
| 166,1g | VE-Wasser |
| 20,0g | Luviskol®K90 (BASF SE) |
| 0,2g | Parfümöl "Lisa" (Drom Fragrances) |
| 0,8g | Cremophor®CO 40 (BASF SE) |
| 1,0g | Euxyl®PE 9010 (Schülke + Mayr GMBH) |

Diese Formulierung wurde unter Rühren mit Triethanolamin auf pH 7.0 eingestellt.

| | |
|---|---|
| Biegetest : | 212 cN |
| Viskosität: | 66000 m Pas |

### Vergleichsbeispiel

Gleichzeitige Polymerisation von Methacrylamid, Ethylacrylat, Methacrylsäure und eines Methacrylsäureester eines ethoxylierten C16 - C18-Fettalkoholgemisches

Der Versuchsaufbau bestand aus einem 2-L-Reaktionsgefäß mit Ankerrührer, Rückflußkühler und Zulaufgefäßen.

In der Vorlage wurden 360,0g VE-Wasser, 0,6g Natriumdodecylsulfat, 6.0g Methacrylsäure und 9,0g Ethylacrylat unter Rühren (120Upm) auf 80°C aufgeheizt.

Bei Erreichen von 80°C wurden 24,0g einer 2,5 Gew.-%igen wässrigen Natriumperoxodisulfatlösung für den Reaktionsstart schnell zugegeben.

5min nach der Zugabe wurde eine gerührte Emulsion bestehend aus:

| | |
|---|---|
| 40,0g | VE-Wasser |
| 1,65g | Natriumdodecylsulfat |
| 7,2g | Sorbitantristearat-20 EO (Tween®80, Fa.Croda) |
| 25.0g | Methacrylsäureester eines ethoxylierten C16 - C18-Fettalkoholgemisches (Visiomer®C18 PEG-1105-MA 60%ig, Fa. Evonik) |
| 200,0g | Methacrylamid (15%ig in Wasser) |
| 109,0g | Methacrylsäure |
| 126,0g | Ethylacrylat |

in 1,5h in das Reaktionsgefäß zudosiert.

Nach Zulaufende rührte der Versuch noch 2h bei 80°C nach.

Anschließend wurde der Versuch auf Raumtemperatur abgekühlt. Beim Erreichen von 40°C während des Abkühlprozesses erfolgte die Zugabe von 0,9g einer 1 Gew.-%igen wässrigen Wasserstoffperoxidlösung.

5min nach dieser Zugabe wurden 30,0g wässrige 0,25 Gew.-%ige L(+)-Ascorbinsäure in 30min zudosiert.

Die Dispersion wurde dann über einen 120µm Filter abfiltriert.

Der pH-Wert der 31,6%igen Dispersion lag bei ca. 2,9

Mit dieser Dispersion wurde ein Gel formuliert mit einem Anteil von 1,5 Gew.% der Dispersion (nur Feststoffanteil gerechnet)

| | |
|---|---|
| 9,5g | Dispersion Vergleichsbeispiel |
| 166,4g | VE-Wasser |
| 20,0g | Luviskol®K90 (BASF SE) |
| 0,2g | Parfümöl "Lisa" (Drom Fragrances) |
| 0,8g | Cremophor®CO 40 (BASF SE) |
| 1,0g | Euxyl®PE 9010 (Schülke + Mayr) |

Diese Formulierung wurde unter Rühren mit Triethanolamin auf pH 7.0 eingestellt.

| | |
|---|---|
| Biegetest1: | 167 cN |
| Viskosität2: | 67700 mPas |

### Ergebnis:

| | Beispiel 1 | Beispiel 2 | Vergleichsbeispiel |
|---|---|---|---|
| Biegesteifigkeit [cN] | 209 cN | 212 | 167 |
| Verdickungsleistung [mPa*s] | 45500 | 66000 | 67700 |

Eine für Gelformulierungen ausreichende Verdickungsleistung beträgt mindestens 40000 mPa*s, der Wert für die Biegesteifigkeit soll wenigstens 190 cN betragen.

### Haargele

Die im Folgenden dargestellten erfindungsgemäßen Haargele werden wie folgt hergestellt: Zunächst werden die Komponenten der Phase A solubilisiert. Dann wird Phase B angesetzt und gelöst, anschließend in Phase A gegeben und unter Rühren eine gemeinsame Lösung hergestellt. Schließlich wird Phase C in Phase A+B gegeben und bis zur Homogenität gerührt.

Die angegebenen Mengen sind in Gew.-% sofern nicht ausdrücklich anders beschrieben. Dispersion 1 ist die filtierte Polymerdispersion des erfindungsgemäßen Beispiels 1. Anstelle von Dispersion 1 kann in allen folgenden Rezepturen jede beliebige erfindungsgemäße Polymerdispersion eingesetzt werden, insbesondere auch die Polymerdispersion des erfindungsgemäßen Beispiels 2.

### Haargel 1

| Ingredient | INCl (Hersteller) |
|---|---|
| Phase A | |
| 0,40 Cremophor^{®} CO40 | PEG-40 |
| Hydrogenated Castor Oil | |
| 0,10 Parfümöl | |
| 81,09 Wasser dest. | Aqua dem. |
| | |

| Phase B | |
|---|---|
| 15,00 Luviskol^{®} K90 (3% Polymergehalt) | PVP |
| 2,57 Dispersion 1 (0,8% WS) | |
| 0,50 Euxyl^{®} PE 9010 | Phenoxyethanol Ethylhexyl Glycerin |
| | |

| Phase C | |
|---|---|
| 0,34 AMP | 2-Amino-2-Methyl-propanol |

### Haargel 2

| Ingredient | INCI (Hersteller) |
|---|---|
| Phase A | |
| 0,40 Cremophor^{®} CO40 | PEG-40Hydrogenated Castor Oil |
| 0,10 Parfümöl | |
| 85,45 Wasser dest. | Aqua dem. |
| | |

| Phase B | |
|---|---|
| 10,00 Luviskol^{®} VA64W (5% Polymergehalt) | PVPNA |
| 3,21 Dispersion 1 (1,0% Polymergehalt) | |
| 0,50 Euxyl^{®} PE 9010 | Phenoxyethanol Ethylhexyl Glycerin |
| | |

| Phase C | |
|---|---|
| 0,34 AMP | 2-Amino-2Methyl-propanol |

### Haargel 3

| Ingredient | INCI (Hersteller) |
|---|---|
| Phase A | |
| 0,40 Cremophor^{®} CO40 | PEG-40Hydrogenated Castor Oil |
| 0,10 Parfümöl | |
| 80,45 Wasser dest. | Aqua dem. |

| Phase B | |
|---|---|
| 15,00 Luviset^{®} Clear (3% Polymergehalt) | VP/Methacrylamide/N-Vinyl Imidazole Polymer |
| 3,21 Dispersion 1 (1,0% Polymergehalt) | |
| 0,50 Euxyl^{®} PE 9010 | Phenoxyethanol Ethylhexyl Glycerin |
| | |

| Phase C | |
|---|---|
| 0,34 AMP | 2-Amino-2Methyl-propanol |

Haargel 4

| Ingredient | INCI (Hersteller) |
|---|---|
| Phase A | |
| 0,40 Cremophor^{®} CO40 | PEG-40Hydrogenated Castor Oil |
| 0,10 Parfümöl | |
| 85,45 Wasser dest. | Aqua dem. |
| | |

| Phase B | |
|---|---|
| 2,50 Luviquat^{®} Supreme (0,5% Polymergehalt) | Polyquaternium-68 |
| 7,50 Luviset^{®} Clear (1,50% Polymergehalt) | VP/Methacrylamide/N-Vinyl Imidazole Polymer |
| 3,21 Dispersion 1 (1,0% Polymergehalt) | |
| 0,50 Euxyl^{®} PE 9010 | Phenoxyethanol Ethylhexyl Glycerin |
| | |

| Phase C | |
|---|---|
| 0,34 AMP | 2-Amino-2Methyl-propanol |

### Haargel 5

| Ingredient | INCI (Hersteller) |
|---|---|
| Phase A | |
| 0,40 Cremophor^{®} CO40 | PEG-40 Hydrogenated Castor Oil |
| 0,10 Parfümöl | |
| 77,95 Wasser dest. | Aqua dem. |
| | |

| Phase B | |
|---|---|
| 2,50 Luviquat^{®} Supreme (0,5% Polymergehalt) | Polyquaternium-68 |
| 15,00 Luviskol^{®} K90 (3,0% Polymergehalt) | PVP |
| 3,21 Dispersion 1 (1,0% Polymergehalt) | |
| 0,50 Euxyl^{®} PE 9010 | Phenoxyethanol Ethylhexyl Glycerin |
| | |

| Phase C | |
|---|---|
| 0,34 AMP | 2-Amino-2-Methyl-propanol |

### Haargel 6

| Ingredient | INCl (Hersteller) |
|---|---|
| Phase A | |
| 0,40 Cremophor^{®} CO40 | PEG-40 Hydrogenated Castor Oil |
| 0,10 Parfümöl | |
| 82,95 Wasser dest. | Aqua dem. |
| | |

| Phase B | |
|---|---|
| 2,50 Luviquat^{®} Supreme (0,5% Polymergehalt) | Polyquaternium-68 |
| 1,00 Luviskol^{®} VA64 (5,0% Polymergehalt) | PVP/VA |
| 3,21 Dispersion 1 (1,0% Polymergehalt) | |
| 0,50 Euxyl^{®} PE 9010 | Phenoxyethanol Ethylhexyl Glycerin |
| | |

| Phase C | |
|---|---|
| 0,34 AMP | Amino-2-Methyl-propanol |

### Haargel 7

| Ingredient | INCI (Hersteller) |
|---|---|
| Phase A | |
| 0,30 Cremophor^{®} CO40 | PEG-40 Hydrogenated Castor Oil |
| 0,10 Parfümöl | |
| 79,84 Wasser dest. | Aqua dem. |
| | |

| Phase B | |
|---|---|
| 10,00 Luviset^{®} Clear (2,0% Polymergehalt) | VP/Methacrylamide/N-Vinyl Imidazole Polymer |
| 3,33 Dispersion 1 (1,0% Polymergehalt) | |
| 2,50 Karion^{®} F | Sorbitol |
| 2,50 1,2 Propylenglykol Care | Propylenglykol |
| 0,50 Panthenol 50P | Panthenol |
| 0,10 Niacinamid | Niacinamid (Nutrilo) |
| 0,50 Euxyl^{®}PE 9010 | Phenoxyethanol Ethylhexyl Glycerin |
| | |

| Phase C | |
|---|---|
| 0,33 AMP | 2-Amino-2Methyl-propanol |

### Haargel 8

| Ingredient | INCI (Hersteller) |
|---|---|
| Phase A | |
| 0,30 Cremophor^{®} CO40 | PEG-40Hydrogenated Castor Oil |
| 0,10 Parfümöl | |
| 74,84 Wasser dest. | Aqua dem. |
| | |

| Phase B | |
|---|---|
| 5,00 Luviskol^{®}VA 64W (2,5% Polymergehalt) | PVP/VA Copolymer |
| 10,00 Luviset^{®}Clear (2,0% Polymergehalt) | VP/Methacrylamide/N-Viny Imidazole Polymer |
| 3,33 Dispersion 1 (1,0% Polymergehalt) | |
| 2,50 Karion^{®}F | Sorbitol |
| 2,50 1,2 Propylenglykol Care | Propylenglykol |
| 0,50 Panthenol 50P | Panthenol |
| 0,10 Niacinamid | Niacinamid (Nutrilo) |
| 0,50 Euxyl^{®}PE 9010 | Phenoxyethanol Ethylhexyl Glycerin |
| | |

| Phase C | |
|---|---|
| 0,33 AMP | 2-Amino-2Methyl-propanol |

### Haarshampoos

Die im Folgenden angegebenen Mengen sind in Gew.-% sofern nicht ausdrücklich anders beschrieben. Dispersion 1 ist die filtierte Polymerdispersion des erfindungsgemäßen Beispiels 1.

### Conditioning Shampoo mit Jaguar® C 13 S und Siliconen

| Ingredient | INCI (Hersteller) |
|---|---|
| Phase A | |
| 0,20 Jaguar^{®}C 13 S | Guar Hydroxypropyltrimonium Chloride |
| 62,00 Wasser dest. | Aqua dem. |
| | |

| Phase B | |
|---|---|
| 17,50 Texapon^{®}N 701 (12%) | Sodium laureth Sulfate |
| 5,40 Tego^{®}Betain L7 (1,6%) | Cocoamidopropyl Betaine (Evonik) |
| | |

| Phase C | |
|---|---|
| 1,60 Dispersion 1 | |
| q.s. NaOH 20% | Sodium Hydroxide |
| | |

| Phase D | |
|---|---|
| 2,20 Dow Corning^{®}Dispersion 1785 (1,3%) | Dimethiconol, TEA-Dodecylbenzenesulfonate |
| 1,10 Dow Corning^{®}Emulsion CE-8170 (0,2%) | Amodimethocone, C11-15 Pareth-7, Glycerin, Trideceth-12 |
| 9,10 Euperlan^{®}3000 AM (2%) | Glycol Distearate, Laureth-4, Cocamidopropyl Betaine |
| 0,20 Parfümöl | |
| 0,20 Glydant^{®} LTD. | DMDM Hydantoin |
| | |

| Phase E | |
|---|---|
| q.s. Citronensäure | Citric Acid |
| 0,50 Natriumchlorid | Natriumchlorid |

### Herstellung:

Die Komponenten der Phase A werden gelöst. Phase B wird angesetzt und in Phase A gegeben. Phase C wird in Phase A+B gegeben und der pH-Wert unter Rühren auf ungefähr 6,7 eingestellt. Phase D wird in die Mischung A+B+C homogen eingerührt.

Der pH-Wert der erhaltenen Mischung wird mit Citronensäure auf ungefähr pH 6,0 eingestellt, Natriumchlorid wird zugegeben und alles homogen gerührt.

### Conditioning Shampoo mit UCARE^{®} Polymer 400C und Siliconen

| Ingredient | INCI (Hersteller) |
|---|---|
| Phase A | |
| 0,20 UCARE^{®} Polymer JR 400 | Polyquaternium-10 |
| 62,00 Wasser dest. | Aqua dem. |

| Phase B | |
|---|---|
| 17,50 Texapon^{®}N 701 (12%) | Sodium laureth Sulfate |
| 5,40 Tego^{®}Betain L7 (1,6%) | Cocoamidopropyl Betaine (Evonik) |
| | |

| Phase C | |
|---|---|
| 1,60 Dispersion 1 | |
| q.s. NaOH 20% | Sodium Hydroxide |
| | |

| Phase D | |
|---|---|
| 2,20 Dow Corning^{®}Dispersion 1785 (1,3%) | Dimethiconol, TEA-Dodecylbenzenesulfonate |
| 1,10 Dow Corning^{®}Emulsion CE-8170 (0,2%) | Amodimethocone, C11-15 Pareth-7, Glycerin, Trideceth-12 |
| 9,10 Euperlan^{®}3000 AM (2%) | Glycol Distearate, Laureth-4, Cocamidopropyl Betaine |
| 0,20 Parfümöl | |
| 0,20 Glydant^{®} LTD. | DMDM Hydantoin |
| | |

| Phase E | |
|---|---|
| q.s. Citronensäure | Citric Acid |
| 0,50 Natriumchlorid | Natriumchlorid |

### Herstellung:

Die Komponenten der Phase A werden gelöst. Phase B wird angesetzt und in Phase A gegeben. Phase C wird in Phase A+B gegeben und der pH-Wert unter Rühren auf ungefähr 6,7 eingestellt. Phase D wird in die Mischung A+B+C homogen eingerührt.

Der pH-Wert der erhaltenen Mischung wird mit Citronensäure auf ungefähr pH 6,0 eingestellt, Natriumchlorid wird zugegeben und alles homogen gerührt.

### Conditioning Shampoo mit geringerem Tensidanteil

| Ingredient | INCI (Hersteller) |
|---|---|
| Phase A | |
| 3,30 Dispersion 1 | |
| 59,80 Wasser dest. | Aqua dem. |
| q.s. NaOH 20% | Sodium Hydroxide |
| | |

| Phase B | |
|---|---|
| 0,10 Salcare^{®}CS 60 | Acrylamidopropyltrimonium Chloride |
| 2,90 Wasser dest. | Aqua dem. |

| Phase C | |
|---|---|
| 25,80 Texapon^{®}NSO (7% Tensidanteil) | Sodium Laureth Sulfate |
| 6,80 Tego^{®}Betain L7 (2,0% Tensidanteil) | Cocoamidopropyl Betaine |
| 0,30 Parfüm | |
| q.s. Konservierungsmittel | |
| | |

| Phase D | |
|---|---|
| q.s. Citronensäure | Citric Acid |
| 0,50 Natriumchlorid | Natriumchlorid |

### Herstellung:

Die Komponenten der Phase A werden eingewogen und gelöst, anschließend mit NaOH ungefähr auf pH 6,7 eingestellt. Die Phase B wird eingewogen und gelöst, anschließend in Phase A gegeben. Die Komponenten der Phase C werden in Phase A+B gegeben und homogen gerührt. Anschließend wird der pH-Wert mit Citronensäure auf ungefähr pH 6,0 eingestellt, Natriumchlorid zugegeben und das Gemisch homogen gerührt.

### Anti-Schuppen-Shampoo mit Zink Pyrithione

| Ingredient | INCI (Hersteller) |
|---|---|
| Phase A | |
| 3,20 Dispersion 1 | |
| 41,00 Wasser dest. | Aqua dem. |
| q.s. NaOH 20% | Sodium Hydroxide |
| | |

| Phase B | |
|---|---|
| 0,80 Luviquat^{®}Sensation (0,2% Polymeranteil) | Polyquaternium-87 |
| 2,90 Wasser dest. | Aqua dem. |

| Phase C | |
|---|---|
| 35,70 Texapon^{®}NSO (9,7% Tensidanteil) | Sodium Laureth Sulfate |
| 12,50 Tego^{®}Betain L7 (3,7% Tensidanteil) | Cocoamidopropyl Betaine |
| 2,50 Zink-Pyrion^{®} 48% Micro (1,2% Aktivanteil) | Zink Pyrithione |
| | |

| Phase D | |
|---|---|
| 0,30 Parfüm | |
| q.s. Konservierungsmittel | |
| | |

| Phase E | |
|---|---|
| q.s Citronensäure | Citronensäure |

### Herstellung:

Die Komponenten der Phase A werden eingewogen und gelöst, anschliessend mit NaOH ungefähr auf pH 6,7 eingestellt. Die Phase B wird eingewogen und gelöst, anschliessend in Phase A gegeben. Die Komponenten der Phase C werden in Phase A+B gegeben und homogen gerührt. Anschliessend wird Phase D zugegeben und die erhaltene Mischung homogenisiert. Schließlich wird der pH-Wert mit Citronensäure auf ungefähr pH 6,0 eingestellt.

### Conditioning Shampoo mit Luviquat^{®}Sensation, Uvinul^{®}MC 80

| Ingredient | INCI (Hersteller) |
|---|---|
| Phase A | |
| 2,00 Luviquat^{®}Sensation (0,5% Polymeranteil) | Polyquaternium-87 |
| 48,30 Wasser dest. | Aqua dem. |
| q.s. Konservierungsmittel | Preservative |
| | |

| Phase B | |
|---|---|
| 8,90 Dehyton^{®}PK 45 | Cocamidopropyl Betaine |
| 35,70 Texapon^{®}NSO (9,7% Tensidanteil) | Sodium Laureth Sulfate |
| | |

| Phase C | |
|---|---|
| 1,60 Dispersion 1 | |
| q.s. NaOH 20% | Sodium Hydroxide |
| | |

| Phase D | |
|---|---|
| 0,50 D-Panthenol^{®}USP | Panthenol |
| 2,00 Uvinul^{®}MC 80 | Ethylhexyl Methoxycinnamate |
| 0,30 Parfümöl | Perfume |
| 0,10 Edeta BD | Disodium EDTA |
| | |

| Phase E | |
|---|---|
| 0,70 Natriumchlorid | Sodium Chloride |

### Herstellung:

Die Komponenten der Phase A werden eingewogen und gemischt. Phase B wird nacheinander in Phase A gegeben und gelöst. Die Komponenten der Phase C werden der Phase A+B zugegeben, der pH-Wert auf ca. pH 6 eingestellt und bis zur Homogenität gerührt. Phase D wird zugegeben und gelöst. Die Viskosität wird mit Phase E eingestellt.

### Conditioning Shampoo mit Salcare^{®}SC 60

| Ingredient | INCI (Hersteller) |
|---|---|
| Phase A | |
| 0,10 Salcare^{®}SC 60 | Acrylamidepropyltrimonium Chloride, Acrylamide |
| 15,00 Wasser dest. | Aqua dem. |
| q.s. Konservierungsmittel | Preservative |
| | |

| Phase B | |
|---|---|
| 1,60 Dispersion 1 | |
| 33,30 Wasser dest. | Aqua dem. |
| q.s. NaOH 20% | Sodium Hydroxide |
| | |

| Phase C | |
|---|---|
| 8,90 Dehyton^{®}PK 45 | Cocamidopropyl Betaine |
| 35,70 Texapon^{®}NSO (9,7% Tensidanteil) | Sodium Laureth Sulfate |
| | |

| Phase D | |
|---|---|
| 0,50 D-Panthenol^{®}USP | Panthenol |
| 4,00 Dow Coming^{®}1664 | Dimethicone, Laureth-4, Laureth-23 |
| 0,30 Parfümöl | Perfume |
| | |

| Phase E | |
|---|---|
| 0,50 Natriumchlorid | Sodium Chloride |

### Herstellung:

Die Komponenten der Phase A werden eingewogen und gelöst. Phase B wird eingewogen und mit NaOH neutralisiert. Phase C wird in Phase B gegeben und homogen gerührt. Danach wird die gelöste Phase A in Phase B+C gegeben und homogen gerührt. Der pH-Wert wird auf ca. pH=6 eingestellt. Phase D wird zugegeben und gelöst. Mit Phase E wird die Viskosität eingestellt.

### Haarfarben-Formulierung - Entwickler

| Ingredient | INCI (Hersteller) |
|---|---|
| Phase A | |
| 80,00 Wasser dest. | Aqua dem. |
| 0,15 Disodium Phosphate | Disodium Phosphate |

| Phase B | |
|---|---|
| 5,00 Dispersion 1 | |
| | |

| Phase C | |
|---|---|
| 12,00 Wasserstoffperoxid (50%) | Hydrogen Peroxid |
| 1,00 Glycerin (99%) | Glycerin |
| 1,00 Texapon^{®}NSO (0,27% Tensidanteil) | Sodium Laureth Sulfate |
| | |

| Phase D | |
|---|---|
| q.s. Sequestrante HEDP | Etidronic Acid |

### Herstellung:

Die Komponenten der Phase A werden eingewogen und gelöst. Anschliessend werden die Komponenten der Phasen B und C in Phase A gegeben und gerührt. Mit Phase D wird der pH-Wert auf 2,5 bis 3 eingestellt.

## Patentansprüche

1. Verfahren zur Herstellung von Polymerisaten P umfassend ein Gemisch aus wenigstens einem Polymer A und wenigstens einem Polymer B, wobei
A) Polymer A in einpolymerisierter Form umfasst
a1) 30 bis 70 Gew.-% C₁-C₄-Alkyl-(Meth)acrylat,
a2) 30 bis 70 Gew.-% (Meth)Acrylsäure,
a3) 0 bis 20 Gew.-% mit einem C₈-C₃₀-Alkyl-substituierte Monomere,
a4) 0 bis 20 Gew.-% weitere von a1) bis a3) verschiedene Monomere,
wobei sich die Mengen von a1) bis a4) zu 100 Gew.-% addieren,
und
B) Polymer B in einpolymerisierter Form umfasst
b1) wenigstens ein Monomer b1), das wenigstens eine Amidgruppe trägt, und
b2) gegebenenfalls weitere von b1) verschiedene Monomere b2)
**dadurch gekennzeichnet, dass** die Monomere eines der Polymere A oder B teilweise oder nahezu vollständig polymerisiert werden und dann die Monomere des jeweils anderen Polymers hinzugegeben werden, wonach anschließend das jeweils andere Polymer und das teilweise polymerisierte Polymer A oder B oder das nahezu vollständig polymerisierte Polymer A oder B unter Bildung des Gemischs aus Polymer A und Polymer B auspolymerisiert werden.

2. Verfahren nach Anspruch 1, **dadurch gekennzeichnet, dass** die Monomere des jeweils anderen Polymers erst dann hinzugegeben werden, wenn mehr als 99 Gew.-% der Menge aller zur Polymerisation von Polymer A oder Polymer B einzusetzenden Monomere polymerisiert sind.

3. Verfahren nach Anspruch 2, **dadurch gekennzeichnet, dass** die Monomere b1) und b2) erst dann hinzugegeben werden, wenn mehr als 99 Gew.-% der Menge aller zur Polymerisation des Polymers A einzusetzenden Monomere a1) bis a4) polymerisiert sind.

4. Verfahren nach Anspruch 1, **dadurch gekennzeichnet, dass** die Monomere des jeweils anderen Polymers dann hinzugegeben werden, wenn im Bereich von 50 bis 99 Gew.-% aller zur Polymerisation von Polymer A oder Polymer B einzusetzenden Monomere polymerisiert sind.

5. Verfahren nach Anspruch 1, **dadurch gekennzeichnet, dass** die Monomere des jeweils anderen Polymers hinzugegeben werden, wenn im Bereich von 10 bis 50 Gew.-% aller zur Polymerisation von Polymer A oder Polymer B einzusetzenden Monomere polymerisiert sind und beendet wird, bevor mehr als 80 Gew.-% der Menge aller zur Polymerisation von Polymer A oder Polymer B einzusetzenden Monomere polymerisiert sind.

6. Verfahren nach einem der Ansprüche 1 bis 5, **dadurch gekennzeichnet, dass** die Herstellung der Polymere A und B durch radikalische Emulsionspolymerisation erfolgt.

7. Verfahren nach einem der Ansprüche 1 bis 6, **dadurch gekennzeichnet, dass** das Polymerisat P die Polymere A und B in einem Gewichtsverhältnis A:B im Bereich von 50:50 bis 98:2 umfasst.

8. Verfahren nach einem der Ansprüche 1 bis 7, **dadurch gekennzeichnet, dass** wenigstens ein Monomer b1) Methacrylamid ist.

9. Verfahren nach einem der Ansprüche 1 bis 8, **dadurch gekennzeichnet, dass** a1) Ethylacrylat ist oder umfasst und a2) Methacrylsäure ist oder umfasst.

10. Polymerisat P erhältlich nach einem Verfahren nach einem der Ansprüche 1 bis 9.

11. Kosmetische Zubereitung enthaltend ein Polymerisat P gemäß Anspruch 10.

12. Verwendung eines Polymerisates P gemäß Anspruch 10 oder eines nach den Ansprüchen 1 bis 9 hergestellten Polymerisats P als Verdickungsmittel.

13. Verfahren zur Behandlung von Haaren **dadurch gekennzeichnet, dass** die Haare mit einem Polymerisat P gemäß Anspruch 10 in Kontakt gebracht werden.

## Claims

1. A process for the preparation of polymers P comprising a mixture of at least one polymer A and at least one polymer B, where
polymer A comprises, in polymerized-in form
30 to 70% by weight of C₁-C₄-alkyl (meth)acrylate,
30 to 70% by weight of (meth)acrylic acid,
0 to 20% by weight of monomers substituted with a C₈-C₃₀-alkyl,
0 to 20% by weight of further monomers different from a1) to a3), where the amounts of a1) to a4) add up to 100% by weight, and
polymer B comprises, in polymerized-in form,
at least one monomer b1) which carries sat least one amide group, and
optionally further monomers b2) different from b1)
wherein the monomers of one of the polymers A or B are partially or virtually completely polymerized and then the monomers of the other polymer in each case are added, after which the other polymer in each case and the partially polymerized polymer A or B or the virtually completely polymerized polymer A or B are then fully polymerized to form the mixture of polymer A and polymer B.

2. The process according to claim 1, wherein the monomers of the other polymer in each case are only added when more than 99% by weight of the amount of all monomers to be used for the polymerization of polymer A or polymer B have been polymerized.

3. The process according to claim 2, wherein the monomers b1) and b2) are only added when more than 99% by weight of the amount of all monomers a1) to a4) to be used for the polymerization of polymer A have been polymerized.

4. The process according to claim 1, wherein the monomers of the other polymer in each case are added when in the region of from 50 to 99% by weight of all monomers to be used for the polymerization of polymer A or polymer B have been polymerized.

5. The process according to claim 1, wherein the monomers of the other polymer in each case are added when in the region of from 10 to 50% by weight of all monomers to be used for the polymerization of polymer A or polymer B have been polymerized and is ended before more than 80% by weight of the amount of all monomers to be used for the polymerization of polymer A or polymer B have been polymerized.

6. The process according to any one of claims 1 to 5, wherein the preparation of the polymers A and B takes place by radical emulsion polymerization.

7. The process according to any one of claims 1 to 6, wherein the polymer P comprises the polymers A and B in a weight ratio A:B in the range from 50:50 to 98:2.

8. The process according to any one of claims 1 to 7, wherein at least one monomer b1) is methacrylamide.

9. The process according to any one of claims 1 to 8, wherein a1) is or comprises ethyl acrylate and a2) is or comprises methacrylic acid.

10. A polymer P obtainable by a process according to any one of claims 1 to 9.

11. A cosmetic preparation comprising a polymer P according to claim 10.

12. The use of a polymer P according to claim 10 or of a polymer P prepared according to claims 1 to 9 as thickener.

13. A method of treating hair, wherein the hair is brought into contact with a polymer P according to claim 10.

## Revendications

1. Procédé pour la préparation de polymérisats P, comprenant un mélange d'au moins un polymère A et d'au moins un polymère B,
A) le polymère A comprenant sous forme incorporée par polymérisation
a1) 30 à 70 % en poids de (méth)acrylate d'alkyle en C₁-C₄,
a2) 30 à 70 % en poids d'acide (méth)acrylique,
a3) 0 à 20 % en poids de monomères substitués par alkyle en C₈-C₃₀,
a4) 0 à 20 % en poids d'autres monomères différents de a1) à a3),
la somme des quantités de a1) à a4) étant égale à 100 % en poids, et
B) le polymère B comprenant sous forme incorporée par polymérisation
b1) au moins un monomère b1) qui porte au moins un groupe amido, et
b2) éventuellement d'autres monomères b2) différents de b1)
**caractérisé en ce qu'**on fait polymériser en partie ou en presque totalité les monomères de l'un des polymères A ou B et ensuite on ajoute les monomères de l'autre polymère respectif, à la suite de quoi on fait ensuite polymériser complètement l'autre polymère respectif et le polymère A ou B en partie polymérisé ou le polymère A ou B en presque totalité polymérisé, avec formation du mélange de polymère A et polymère B.

2. Procédé selon la revendication 1, **caractérisé en ce qu'**on n'ajoute ensuite les monomères de l'autre polymère respectif que lorsque plus de 99 % en poids de la quantité de tous les monomères à utiliser dans la polymérisation du polymère A ou du polymère B sont polymérisés.

3. Procédé selon la revendication 2, **caractérisé en ce qu'**on n'ajoute ensuite les monomères b1) et b2) que lorsque plus de 99 % en poids de la quantité de tous les monomères a1) à a4) à utiliser dans la polymérisation du polymère A sont polymérisés.

4. Procédé selon la revendication 1, **caractérisé en ce qu'**on ajoute ensuite les monomères de l'autre polymère respectif lorsque dans la plage de 50 à 99 % en poids de tous les monomères à utiliser dans la polymérisation du polymère A ou du polymère B sont polymérisés.

5. Procédé selon la revendication 1, **caractérisé en ce qu'**on ajoute les monomères de l'autre polymère respectif lorsque dans la plage de 10 à 50 % en poids de tous les monomères à utiliser dans la polymérisation du polymère A ou du polymère B sont polymérisés et on termine avant que plus de 80 % en poids de la quantité de tous les monomères à utiliser dans la polymérisation du polymère A ou du polymère B soient polymérisés.

6. Procédé selon l'une quelconque des revendications 1 à 5, **caractérisé en ce que** la préparation des polymères A et B s'effectue par polymérisation radicalaire en émulsion.

7. Procédé selon l'une quelconque des revendications 1 à 6, **caractérisé en ce que** le polymérisat P comprend les polymères A et B en un rapport pondéral A:B dans la plage de 50:50 à 98:2.

8. Procédé selon l'une quelconque des revendications 1 à 7, **caractérisé en ce qu'**au moins un monomère b1) est le méthacrylamide.

9. Procédé selon l'une quelconque des revendications 1 à 8, **caractérisé en ce que** a1) est ou comprend l'acrylate d'éthyle et a2) est ou comprend l'acide méthacrylique.

10. Polymérisat P pouvant être obtenu conformément à un procédé selon l'une quelconque des revendications 1 à 9.

11. Préparation cosmétique contenant un polymérisat P selon la revendication 10.

12. Utilisation d'un polymérisat P selon la revendication 10 ou d'un polymérisat P préparé selon les revendications 1 à 9 en tant qu'épaississant.

13. Procédé pour le traitement des cheveux, **caractérisé en ce qu'**on met les cheveux en contact avec un polymérisat P selon la revendication 10.
